(19) Europäisches Patentamt
European Patent Office
Office européen des brevets

(11) **EP 3 236 262 B1**

(12) **EUROPEAN PATENT SPECIFICATION**

(45) Date of publication and mention
of the grant of the patent:
**25.09.2019 Bulletin 2019/39**

(51) Int Cl.:
***G01N 33/574*** *(2006.01)*

(21) Application number: **17159039.1**

(22) Date of filing: **10.05.2013**

(54) **SURROGATE FUNCTIONAL DIAGNOSTICS TEST FOR CANCER**

SURROGATFUNKTIONSDIAGNOSETEST FÜR KREBS

ESSAI DE DIAGNOSTIC FONCTIONNEL DE SUBSTITUT POUR LE CANCER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.05.2012 US 201261645253 P**
**13.03.2013 US 201361780252 P**

(43) Date of publication of application:
**25.10.2017 Bulletin 2017/43**

(62) Document number(s) of the earlier application(s) in accordance with Art. 76 EPC:
**13787323.8 / 2 847 592**

(73) Proprietor: **Eutropics Pharmaceuticals, Inc.**
**Cambridge, Massachusetts 02138 (US)**

(72) Inventors:
• **PIERCEALL, William**
**Sudbury, MA 01776 (US)**
• **CARDONE, Michael H.**
**Dorchester, MA 02125 (US)**

(74) Representative: **Grund, Martin**
**Grund Intellectual Property Group**
**Patentanwalt und Solicitor PartG mbB**
**Postfach 44 05 16**
**80754 München (DE)**

(56) References cited:
• **MICHAEL THOMENIUS ET AL: "Using BH3 Profiling As a Predictive Indicator for Myeloma Patient Response to Bortezomib e p", BLOOD, vol. 118, no. 21, 18 November 2011 (2011-11-18), page 1690, XP055235506,**

• **FRANK A SINICROPE ET AL: "Proapoptotic Bad and Bid Protein Expression Predict Survival in Stages II and III Colon Cancers", CLINICAL CANCER RESEARCH, THE AMERICAN ASSOCIATION FOR CANCER RESEARCH, US , vol. 14 1 July 2008 (2008-07-01), pages 4128-4133, XP003030690, ISSN: 1078-0432, DOI: 10.1158/1078-0432.CCR-07-5160 Retrieved from the Internet: URL:http://clincancerres.aacrjournals.org/ content/14/13/4128**
• **F. A. SINICROPE ET AL: "Prognostic Impact of Bim, Puma, and Noxa Expression in Human Colon Carcinomas", CLINICAL CANCER RESEARCH, vol. 14, no. 18, 15 September 2008 (2008-09-15), pages 5810-5818, XP055235508, US ISSN: 1078-0432, DOI: 10.1158/1078-0432.CCR-07-5202**
• **T. N. CHONGHAILE ET AL: "Pretreatment Mitochondrial Priming Correlates with Clinical Response to Cytotoxic Chemotherapy", SCIENCE, vol. 334, no. 6059, 27 October 2011 (2011-10-27), pages 1129-1133, XP055093972, ISSN: 0036-8075, DOI: 10.1126/science.1206727 & T. N. CHONGHAILE ET AL: "Supporting online material for "Pretreatment Mitochondrial Priming Correlates with Clinical Response to Cytotoxic Chemotherapy"", SCIENCE, vol. 334, no. 6059, 27 October 2011 (2011-10-27), pages 1129-1133, XP055093976, ISSN: 0036-8075, DOI: 10.1126/science.1206727**

**(Cont. next page)**

- CHONGHAILE TRIONA NI ET AL: "Mitochondrial Apoptotic Priming Measured by BH3 Profiling Regulates Clinical Response to Chemotherapy in Myeloma and Acute Lymphoblastic Leukemia and Explains Therapeutic Index Paper: Mitochondrial Apoptotic Priming Measured by BH3 Profili", BLOOD, 10 December 2011 (2011-12-10), XP055260179,
- L BODET ET AL: "BH3-only protein Bik is involved in both apoptosis induction and sensitivity to oxidative stress in multiple myeloma", BRITISH JOURNAL OF CANCER, vol. 103, no. 12, 9 November 2010 (2010-11-09), pages 1808-1814, XP055182740, ISSN: 0007-0920, DOI: 10.1038/sj.bjc.6605981
- VICTORIA DEL GAIZO MOORE ET AL: "Chronic lymphocytic leukemia requires BCL2 to sequester prodeath BIM, explaining sensitivity to BCL2 antagonist ABT-737", JOURNAL OF CLINICAL INVESTIGATION, vol. 117, no. 1, 2 January 2007 (2007-01-02), pages 112-121, XP055044156, ISSN: 0021-9738, DOI: 10.1172/JCI28281

## Description

### FIELD OF THE INVENTION

The present invention relates to methods that are useful in evaluating tumors in human samples.

### BACKGROUND

The use of predictive and prognostic biomarkers paired with targeted cancer therapies may hold the key to reducing drug development time, improving drug efficacy, and guiding clinical decision making. While there are advances in cancer treatment, chemotherapy remains largely inefficient and ineffective. One reason for the generally poor performance of chemotherapy is that the selected treatment is often not closely matched to the individual patient's disease. A personalized medicine approach that couples precise diagnostics with therapeutics might alleviate this problem.

To date there are only a handful of biomarkers that have added value to clinical oncology practice. In part this is because perceived markers often are correlative but not causal to drug mechanism. Even when the "biomarker" biology does line up with the pharmacology of the companion therapy there is still significant challenge to predicting how a drug will work in a patient. Beyond this, the path to clinical development requires the participation of physician-scientists who see the value of the test and believe it can bring benefit to their patients.

BH3 profiling measures the functionality of a pivotal causal factor to cancer cell response to chemotherapy. Specifically, BH3 profiling measures the functionality of proteins at the surface of the mitochondria that control apoptosis. Many chemotherapies rely on apoptosis to be effective. The readout of the test provides a response of the mitochondria to BH3 domains of the pre-apoptotic BH3 only proteins. While BH3 profiling is known to provide a general sense of chemosensitivity or chemoresponsiveness to therapies, this assay has so far lacked predictive capacity to support physician decision making for certain agents and cancer types.

Thomenius et al. (Blood 2011, 118(21):1690) disclose BH3 profiling of multiple myeloma patients. Sinicrope et al. (Clinical Cancer Research 2008, 15:4128-4133; and Clinical Cancer Research 2008, 14:5810-5818) disclose the prognostic effects of Bad, Bid, Bim, Puma, and Noxa protein expression in colon cancers. Ni Chonghaile et al. (Science 2011, 334(6059):1129-1133; and Blood 2011) disclose that mitochondrial priming by BH3 peptides correlates with clinical response to chemotherapy in different cancers. Bodet et al. (British J. Cancer 2010, 103(12):1808-1814) disclose that Bik protein is involved in apoptosis induction and sensitivity to oxidative stress in multiple myeloma. Del Gaizo Moore et al. (J. Clin. Invest. 2007, 117(1):112-121) disclose that BH3 profiling predicts sensitivity to a BCL2 antagonist in chronic lymphocytic leukemia.

### SUMMARY OF THE INVENTION

Accordingly, the invention provides a method for determining a cancer treatment for a patient, comprising determining a BH3 profile for the patient's cancer cell specimen, wherein the cancer is selected from acute myelogenous leukemia or multiple myeloma, and wherein determining the BH3 profile comprises permeabilizing the patient's cancer cells, determining a change in mitochondrial membrane potential upon contacting the permeabilized cells with one or more BH3 domain peptides, and correlating a loss of mitochondrial membrane potential with chemosensitivity of the cells to apoptosis-inducing chemotherapeutic agents; determining one or more clinical factors of the patient selected from the group consisting of age, cytogenetic status, performance, histological subclass, gender, and disease stage; and classifying the patient for likelihood of clinical response to one or more cancer treatments; wherein the likelihood of the clinical response is determined by assessing a percent priming, wherein the percent priming measures the response of the cancer cell specimen to said contacting with one or more BH3 domain peptides and the percent priming is defined by the following equation:

$$\%Priming = \left[100*\left(\frac{DMSO\ AUC - Peptide_1\ AUC}{DMSO\ AUC - CCCP_{avg}AUC}\right)\right]Peptide_1 + \left[100 * \left(\frac{DMSO\ AUC - Peptide_2\ AUC}{DMSO\ AUC - CCCP_{avg}AUC}\right)\right]Peptide_2 + \cdots / (n\ peptides)$$

wherein:

AUC is either

(i) the area under a curve of a homogenous time-resolved fluorescence (HTRF) measurement, or

(ii) the mean signal intensity of a fluorescence activated cell sorting (FACS) measurement at a single time point that occurs between about 5 min and about 300 min after the start of said contacting, of said change in mitochondrial membrane potential;

DMSO (dimethyl sulfoxide) is a baseline negative control; and

CCCP (Carbonyl cyanide m-chlorophenyl hydrazone) is a chemical inhibitor of oxidative phosphorylation and comprises an effector of protein synthesis by serving as uncoupling agent of the proton gradient established during the normal activity of electron carriers in the electron transport chain in the mitochondria, and is a baseline positive control;

Peptide is said one or more BH3 domain peptides; and

wherein the one or more clinical factors are selected to increase specificity and/or sensitivity of the BH3 profile for association with clinical response.

The details of the invention are set forth in the accompanying description below. In the specification and the appended claims, the singular forms also include the plural unless the context clearly dictates otherwise. Unless defined otherwise, all technical and scientific terms used herein have the same meaning as commonly understood by one of ordinary skill in the art to which this invention belongs.

## BRIEF DESCRIPTION OF THE FIGURES

FIG. 1 shows representative BH3 profiling data. The figure shows differences in patterns of high versus low primed cell lines. MRL-14 is highly primed for BIM 0.3, PUMA 0.3 and NOXA relative to MRL-11 (correlates with therapeutic inhibitor activity by MTS Assay).

FIG. 2 shows representative data for therapeutic inhibitor activity versus traditional growth inhibition EC50 MTS assay.

FIG. 3 shows BH3 profiles for 8 adherent lines. Limited standard deviation among 4-6 replicates for each peptide X cell line

FIG. 4 shows BH3 profiles for 9 suspension lines. Limited standard deviation among 4-6 replicates for each peptide X cell line

FIG. 5 shows BH3 profiling in suspension lines. BIM and BIM_PUMA models discriminate CDKi activity.

FIG. 6 shows BH3 profiling in adherent lines. Models with BIM, PUMA, and NOXA discriminate CDKi activity.

FIG. 7 shows MCL1-inhibitor EC50 versus priming, suspension lines individual peptides.

FIG. 8 shows MCL1-inhibitor EC50 versus priming percentage, suspension lines, multi-peptide -derived algorithms.

FIG. 9 shows MCL1-inhibitor EC50 versus priming percentage, adherent lines, individual peptides.

FIG. 10 shows MCL1-inhibitor versus priming percentage suspension lines, multi-peptide - derived algorithms.

FIG. 11 shows kinesin spindle protein inhibitor (KSP inh), MM, leukemia cell lines.

FIG. 12A-D shows BH3 profiling representative patient data.

FIG. 13A-B shows cytarabine-treated AML patients dot-plot and ROC-plot depictions of BIM patient response discrimination.

FIG. 14 shows cytarabine-treated AML patients BH3 profiling for BH3 Peptides in AML no reposne (NR) and complete response (CR) patients.

**FIG. 15** shows cytarabine-treated AML patients multivariate analysis ROC curve.

**FIG. 16A-H** shows BH3 peptides response prediction stratified by cytogenetic status.

**FIG. 17A-E** shows cytarabine-treated AML patients- correlation of BIM (0.1) priming and BIM (BCL2L11) protein levels and response prediction.

**FIG. 18** shows overall survival (OS) and event free survival (EFS, disease free survival) versus AML patients subgrouped by BIM (0.1) percent priming tertiles.

**FIG. 19.** shows partition analyses of BH3 profiling metrics, individual BH3 peptide models.

**FIG. 20** shows partition analyses of BH3 profiling metrics, combined BH3 peptide models (two peptides).

**FIG. 21** shows partition analyses of BH3 profiling metrics, combined BH3 peptide models (three/four peptides).

**FIG. 22** shows continuous variable analyses of BH3 profiling metrics, individual BH3 peptide models.

**FIG. 23** shows continuous variable analyses of BH3 profiling metrics, combined BH3 peptide models (two peptides).

**FIG. 24** shows continuous variable analyses of BH3 profiling metrics, combined BH3 peptide models (three/four peptides).

**FIG. 25** shows representative AML patients BH3 profiling from azacytidine treatment cohort indicates that the full therapeutic scale of priming values is utilized.

**FIG. 26** shows BIM+NOXA discrimination of azacytidine response in AML patients is superior to either BIM or NOXA independently.

**Table 1** shows a compilation of therapeutic inhibitor response and BH3 profiling by cell line.

**Table 2** shows supporting data summary-MCLI inhibitor.

**Table 3** shows clinicopathologic variables for patient cohort for cytarabine-treated AML patients.

**Table 4** shows cytarabine-treated AML patients. BH3 profiling biomarkers assayed and significance in discriminating response.

**Table 5** shows summary azacytidine efficacy in cell lines, partition and continuous variable models.

## DETAILED DESCRIPTION OF THE INVENTION

The present invention is based, in part, on the discovery that the sensitivity and/or specificity of BH3 profiling measurements can be significantly improved in the context of certain clinical factors. The diagnostic approaches described herein allow for analysis of a suite of BH3 responses and clinical indicators, including ones not directly related to apoptosis, for predicting therapeutic efficacy in human malignancies. For example, the present inventors have discovered that leukemia patient response to cytarabine-based and azacytidine-based chemotherapeutic regimens can be predicted by classifying the patient based on BH3 profiling, age profile and cytogenetic status.

In one aspect, the invention provides a method for determining a cancer treatment for a patient, comprising determining a BH3 profile for the patient's cancer cell specimen; determining one or more clinical factors of the patient, and classifying the patient for likelihood of clinical response to one or more cancer treatments; wherein the one or more clinical factors are selected to increase specificity and/or sensitivity of the BH3 profile for association with clinical response.

The cancer is acute myelogenous leukemia (AML) or multiple myeloma.

In some embodiments, the invention predicts the efficacy of a cancer treatment which can include one or more of anti-cancer drugs, chemotherapy, surgery, adjuvant therapy (*e.g.* prior to surgery), and neoadjuvant therapy (*e.g.* after surgery). In another embodiment, the cancer treatment comprises one or more of a BH3 mimetic, epigenetic modifying agent, topoisomerase inhibitor, cyclin-dependent kinase inhibitor, and kinesin-spindle protein stabilizing agent. In still another embodiment, the cancer treatment comprises a proteasome inhibitor; and/or a modulator of cell cycle regulation (by way of non-limiting example, a cyclin dependent kinase inhibitor); and/or a modulator of cellular epigenetic mechanistic (by way of non-limiting example, one or more of a histone deacetylase (HDAC) (*e.g.* one or more of vorinostat or entinostat), azacytidine, decitabine); and/or an anthracycline or anthracenedione (by way of non-limiting example, one or more of epirubicin, doxorubicin, mitoxantrone, daunorubicin, idarubicin); and/or a platinum-based therapeutic (by way of non-limiting example, one or more of carboplatin, cisplatin, and oxaliplatin); cytarabine or a cytarabine-based chemotherapy; a BH3 mimetic (by way of non-limiting example, one or more of BCL2, BCLXL, or MCL1); and an inhibitor of MCL1.

Determining the BH3 profile comprises permeabilizing the patient's cancer cells, determining a change in mitochondrial membrane potential upon contacting the permeabilized cells with one or more BH3 domain peptides, and correlating the loss of mitochondrial membrane potential with chemosensitivity of the cells to apoptosis-inducing chemotherapeutic agents. These measurements, along with the clinical factors described herein, help differentiate patient response and/or patients for a variety of therapies.

In these or other embodiments, the BH3 profiling comprises use of a peptide, wherein the peptide is one or more of BIM, BIM2A, BAD, BID, HRK, PUMA, NOXA, BMF, BIK, and PUMA2A. In one embodiment, the peptide is used at a concentration of 0.1 μM to 200 μM, and various concentrations therein. The BH3 profiling comprises permeabilizing a specimen to allow access to the mitochondria.

In one embodiment, the specimen is a biopsy selected from a frozen tumor tissue specimen, cultured cells, circulating tumor cells, and a formalin-fixed paraffin-embedded tumor tissue specimen (*e.g.* for antibody based BH3 profiling). In another embodiment, the specimen is a human tumor-derived cell line. In another embodiment, the specimen is a cancer stem cell. In another embodiment, the specimen is derived from the biopsy of a solid tumor (by way of non-limiting example, one or more of colorectal, breast, prostate, lung, pancreatic, renal, or ovarian primary tumor). In another embodiment, the specimen is of epithelial origin, including, for example, an epithelial specimen which is enriched by selection from a biopsy sample with an anti-epithelial cell adhesion molecule (EpCAM) or other epithelial cell binding antibody bound to solid matrix or bead. In another embodiment, the specimen is of mesenchymal origin, including, for example, a mesenchymal specimen which is enriched by selection from a biopsy sample with a neural cell adhesion molecule (N-CAM) or neuropilin or other mesenchymal cell binding antibody bound to a solid matrix or bead. In another embodiment, the specimen is derived from the biopsy of a non-solid tumor. In another embodiment, the specimen is derived from the biopsy of a patient with multiple myeloma, acute myelogenous leukemia, acute lymphocytic leukemia, chronic lymphogenous leukemia, mantle cell lymphoma, diffuse large B-cell lymphoma, and non-Hodgkin's lymphoma. In another embodiment, the specimen is a multiple myeloma cell that is enriched by selection from a biopsy sample with an anti-CD138 antibody bound to a solid matrix or bead. In another embodiment, the cancer cell is an acute myelogenous leukemia that is enriched by binding to a CD45-directed antibody. In yet another embodiment, the cancer cell is a chronic lymphogenous leukemia or diffuse large B-cell lymphoma that is enriched by non-B cell depletion. In another embodiment, the specimen is derived from a circulating tumor cell.

The clinical factor is one or more of age, cytogenetic status, performance, histological subclass, gender, and disease stage.

The likelihodd of the clinical response is determined by assessing a percent priming, wherein the percent priming measures the response of the cancer cell specimen to said contacting with one or more BH3 domain peptides. The percent priming is defined by the following equation:

$$\%Priming = \left[ 100 * \left( \frac{DMSO\ AUC - Peptide_1\ AUC}{DMSO\ AUC - CCCP_{avg}AUC} \right) \right] Peptide_1 + \left[ 100 * \left( \frac{DMSO\ AUC - Peptide_2\ AUC}{DMSO\ AUC - CCCP_{avg}AUC} \right) \right] Peptide_2 + \cdots / (n\ peptides)$$

in which AUC is either area under the curve or mean signal intensity of said change in mitochondrial membrane potential; DMSO is a baseline negative control; and CCCP (Carbonyl cyanide *m*-chlorophenyl hydrazone) is a chemical inhibitor of oxidative phosphorylation and comprises an effector of protein synthesis by serving as uncoupling agent of the proton gradient established during the normal activity of electron carriers in the electron transport chain in the mitochondria and is a baseline positive control. The area under the curve is established by homogenous time-resolved fluorescence (HTRF). In some embodiments, the time occurs over a window from between about 0 to about 300 min to about 0 to about 30 min. The mean signal intensity is that of a fluorescence activated cell sorting (FACS) measurement at a single time point that occurs between about 5 min and about 300 min after the start of said contacting.

In another embodiment, the method comprises conducting the BH3 profiling assay and one or more of a cell surface marker CD33, a cell surface marker CD34, a FLT3 mutation status, a p53 mutation status, a phosphorylation state of MEK-1 kinase, and phosphorylation of serine at position 70 of Bcl-2; and correlating to efficacy in treating AML patients with cytarabine or cytarabine-based chemotherapy and/or azacytidine.

In some embodiments, the cancer is AML and/or MM and the clinical factor is age profile and/or cytogenetic status; or the cancer is AML and/or MM and the cancer treatment is cytarabine or cytarabine-based chemotherapy and/or azacytidine, or the cancer treatment is cytarabine or cytarabine-based chemotherapy and/or azacytidine and the clinical factor is age profile and/or cytogenetic status, or the cancer treatment is cytarabine or cytarabine-based chemotherapy and/or azacytidine; the cancer is AML and/or MM; and the clinical factor is age profile and/or cytogenetic status.

*Exemplary Clinical Decisions*

In various embodiments, the present methods direct a clinical decision regarding whether a patient is to receive a specific treatment. In one embodiment, the present methods are predictive of a positive response to neoadjuvant and/or adjuvant chemotherapy or a non-responsiveness to neoadjuvant and/or adjuvant chemotherapy. In one embodiment, the present methods are predictive of a positive response to a pro-apoptotic agent or an agent that operates via apoptosis and/or an agent that does not operate via apoptosis or a non-responsiveness to apoptotic effector agent and/or an agent that does not operate via apoptosis. In various embodiments, the present invention directs the treatment of a cancer patient, including, for example, what type of treatment should be administered or withheld.

In one embodiment, the present methods direct a clinical decision regarding whether a patient is to receive adjuvant therapy after primary, main or initial treatment, including, without limitation, a single sole adjuvant therapy. Adjuvant therapy, also called adjuvant care, is treatment that is given in addition to the primary, main or initial treatment. By way of non-limiting example, adjuvant therapy may be an additional treatment usually given after surgery where all detectable disease has been removed, but where there remains a statistical risk of relapse due to occult disease.

In some embodiments, the present methods direct a patient's treatment to include adjuvant therapy. For example, a patient that is scored to be responsive to a specific treatment may receive such treatment as adjuvant therapy. Further, the present methods may direct the identity of an adjuvant therapy, by way of non-limiting example, as a treatment that induces and/or operates in a pro-apoptotic manner or one that does not. In one embodiment, the present methods may indicate that a patient will not be or will be less responsive to a specific treatment and therefore such a patient may not receive such treatment as adjuvant therapy. Accordingly, in some embodiments, the present methods provide for providing or withholding adjuvant therapy according to a patient's likely response. In this way, a patient's quality of life, and the cost of care, may be improved.

In various embodiments, the present methods direct a clinical decision regarding whether a patient is to receive neoadjuvant therapy, e.g. therapy to shrink and/or downgrade the tumor prior to surgery. In some embodiments, neoadjuvant therapy means chemotherapy administered to cancer patients prior to surgery. In some embodiments, neoadjuvant therapy means an agent, including those described herein, administered to cancer patients prior to surgery. Types of cancers for which neoadjuvant chemotherapy is commonly considered include, for example, breast, colorectal, ovarian, cervical, bladder, and lung.

In some embodiments, the present methods direct a patient's treatment to include neoadjuvant therapy. For example, a patient that is scored to be responsive to a specific treatment may receive such treatment as neoadjuvant therapy. Further, the present methods may direct the identity of a neoadjuvant therapy, by way of non-limiting example, as a treatment that induces and/or operates in a pro-apoptotic manner or one that does not. In one embodiment, the present methods may indicate that a patient will not be or will be less responsive to a specific treatment and therefore such a patient may not receive such treatment as neoadjuvant therapy. Accordingly, in some embodiments, the present methods provide for providing or withholding neoadjuvant therapy according to a patient's likely response. In this way, a patient's quality of life, and the cost of case, may be improved.

In some embodiments, the present methods direct a clinical decision regarding whether a patient is to receive a specific type of treatment. Accordingly, in some embodiments, the present methods are a guiding test for patient treatment.

In some embodiments, the present methods provide information about the likely response that a patient is to have to a particular treatment. In some embodiments, the present methods provide a high likelihood of response and may direct treatment, including aggressive treatment. In some embodiments, the present methods provide a low likelihood of response and may direct cessation of treatment, including aggressive treatment, and the use of palliative care, to avoid unnecessary toxicity from ineffective chemotherapies for a better quality of life.

In an exemplary embodiment, the present method will indicate a likelihood of response to a specific treatment. For example, in some embodiments, the present methods indicate a high or low likelihood of response to a pro-apoptotic agent and/or an agent that operates via apoptosis and/or an agent that operates via apoptosis driven by direct protein modulation. In various embodiments, exemplary pro-apoptotic agents and/or agents that operate via apoptosis and/or an agent that operates via apoptosis driven by direct protein modulation include ABT-263 (Navitoclax), and obatoclax, WEP, bortezomib, and carfilzomib. In some embodiments, the present methods indicate a high or low likelihood of response to an agent that does not operate via apoptosis and/or an agent that does not operate via apoptosis driven by direct protein modulation. In various embodiments, exemplary agents that do not operate via apoptosis include kinesin spindle protein inhibitors, cyclin-dependent kinase inhibitor, Arsenic Trioxide (TRISENOX), MEK inhibitors, pomolidomide, azacytidine, decitibine, vorinostat, entinostat, dinaciclib, gemtuzumab, BTK inhibitors, PI3 kinase delta inhibitors, lenolidimide, anthracyclines, cytarabine, melphalam, Aky inhibitors, mTOR inhibitors.

In an exemplary embodiment, the present method will indicate whether a patient is to receive a pro-apoptotic agent or an agent that operates via apoptosis for cancer treatment. In another exemplary embodiment, the present method will indicate whether a patient is to receive an agent that does not operate via apoptosis.

In a specific embodiment, the present methods are useful in predicting a cancer patient's response to any of the treatments (including agents) described herein. In an exemplary embodiment, the present invention predicts an AML patient's likelihood of response to cytarabine and azacytidine and comprises an evaluation of the BH3 profile, age profile and cytogenetic factors of the patient.

In various embodiments, a cancer treatment is administered or withheld based on the methods described herein. Exemplary treatments include surgical resection, radiation therapy (including the use of the compounds as described herein as, or in combination with, radiosensitizing agents), chemotherapy, pharmacodynamic therapy, targeted therapy, immunotherapy, and supportive therapy (e.g., painkillers, diuretics, antidiuretics, antivirals, antibiotics, nutritional supplements, anemia therapeutics, blood clotting therapeutics, bone therapeutics, and psychiatric and psychological therapeutics).

*Exemplary Treatments*

Examples of treatment agents include, but are not limited to, one or more of anti-cancer drugs, chemotherapy, surgery, adjuvant therapy, and neoadjuvant therapy. Cancer treatment can be one or more of a BH3 mimetic, epigenetic modifying agent, topoisomerase inhibitor, cyclin-dependent kinase inhibitor, and kinesin-spindle protein stabilizing agent. Cancer treatment can be a proteasome inhibitor; and/or a modulator of cell cycle regulation (by way of non-limiting example, a cyclin dependent kinase inhibitor); and/or a modulator of cellular epigenetic mechanistic (by way of non-limiting example, one or more of a histone deacetylase (HDAC) (*e.g.* one or more of vorinostat or entinostat), azacytidine, decitabine); and/or an anthracycline or anthracenedione (by way of non-limiting example, one or more of epirubicin, doxorubicin, mitoxantrone, daunorubicin, idarubicin); and/or a platinum-based therapeutic (by way of non-limiting example, one or more of carboplatin, cisplatin, and oxaliplatin); cytarabine or a cytarabine-based chemotherapy; a BH3 mimetic (by way of non-limiting example, one or more of BCL2, BCLXL, or MCL1); and an inhibitor of MCL1.

Cancer treatments include, without limitation, those described in US Patent Publication No. US 2012-0225851 and International Patent Publication No. WO 2012/122370.

Cancer treatments include, without limitation, one or more of alkylating agents such as thiotepa and CYTOXAN cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (*e.g.,* bullatacin and bullatacinone); a camptothecin (including the synthetic analogue topotecan); bryostatin; cally statin; CC-1065 (including its adozelesin, carzelesin and bizelesin synthetic analogues); cryptophycins (*e.g.*, cryptophycin 1 and cryptophycin 8); dolastatin; duocarmycin (including the synthetic analogues, KW-2189 and CB 1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (*e.g.*, calicheamicin, especially calicheamicin gammall and calicheamicin omegall (*see, e.g.,* Agnew, Chem. Intl. Ed. Engl., 33: 183-186 (1994)); dynemicin, including dynemicin A; bisphosphonates, such as clodronate; an esperamicin; as well as neocarzinostatin chromophore and related chromoprotein enediyne antiobiotic chromophores), aclacinomysins, actinomycin, authramycin, azaserine, bleomycins, cactinomycin, carabicin, caminomycin, carzinophilin, chromomycinis, dactinomycin, daunorubicin, detorubicin, 6-diazo-5-oxo-L-norleucine, ADRIAMYCIN doxorubicin (including morpholino- doxorubicin, cyanomorpholino-doxorubicin, 2-pyrrolino-doxorubicin and deoxy doxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as minoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; demecolcine; diaziquone; elformithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK polysaccharide complex (JHS Natural Products, Eugene, Oreg.); razoxane; rhizoxin; sizofuran; spirogermanium; tenuazonic acid; triaziquone; 2,2',2"-trichlorotriethylamine; trichothecenes (*e.g.*, T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, *e.g.*, TAXOL paclitaxel (Bristol-Myers Squibb Oncology, Princeton, N.J.), ABRAXANE Cremophor-free, albumin-engineered nanoparticle formulation of paclitaxel (American Pharmaceutical Partners, Schaumberg, 111.), and TAXOTERE doxetaxel (Rhone-Poulenc Rorer, Antony, France); chloranbucil; GEMZAR gemcitabine; 6-thioguanine; mercaptopurine; methotrexate; platinum analogs such as cisplatin, oxaliplatin and carboplatin; vinblastine; platinum; etoposide (VP-16); ifosfamide; mitoxantrone; vincristine; NAVELBINE. vinorelbine; novantrone; teniposide; edatrexate; daunomycin; aminopterin; xeloda; ibandronate; irinotecan (Camptosar, CPT-11) (including the treatment regimen of irinotecan with 5-FU and leucovorin); topoisomerase inhibitor RFS 2000; difluoromethylornithine (DMFO); retinoids such as retinoic acid; capecitabine; combretastatin; leucovorin (LV); oxaliplatin, including the oxaliplatin treatment regimen (FOLFOX); lapatinib (Tykerb); inhibitors of PKC-$\alpha$, Raf, H-Ras, EGFR (*e.g.*, erlotinib (Tarceva)) and VEGF-A that reduce cell proliferation, dacogen, velcade, and pharmaceutically acceptable salts, acids or derivatives of any of the above.

*Exemplary Cancers and Patients*

A cancer or tumor refers to an uncontrolled growth of cells and/or abnormal increased cell survival and/or inhibition of apoptosis which interferes with the normal functioning of the bodily organs and systems. A subject that has a cancer or a tumor is a subject having objectively measurable cancer cells present in the subject's body. Cancers which migrate from their original location and seed vital organs can eventually lead to the death of the subject through the functional deterioration of the affected organs.

In various embodiments, the invention is applicable to pre-metastatic cancer, or metastatic cancer. Metastasis refers to the spread of cancer from its primary site to other places in the body. Cancer cells can break away from a primary tumor, penetrate into lymphatic and blood vessels, circulate through the bloodstream, and grow in a distant focus (metastasize) in normal tissues elsewhere in the body. Metastasis can be local or distant. Metastasis is a sequential process, contingent on tumor cells breaking off from the primary tumor, traveling through the bloodstream, and stopping at a distant site. At the new site, the cells establish a blood supply and can grow to form a life-threatening mass. Both stimulatory and inhibitory molecular pathways within the tumor cell regulate this behavior, and interactions between the tumor cell and host cells in the distant site are also significant. Metastases are often detected through the sole or combined use of magnetic resonance imaging (MRI) scans, computed tomography (CT) scans, blood and platelet counts, liver function studies, chest X-rays and bone scans in addition to the monitoring of specific symptoms.

In one embodiment, the cancer is AML. AML is the second most common leukemia, with approximately 13,000 newly diagnosed cases and 9,000 deaths annually in the US. Although approved therapies exist, the prognosis of many leukemia patients is poor and the likelihood of successful treatment is low. The current standard of care for AML is induction cytosine arabinoside (ara-C) in combination with an anthracycline agent (such as, for example, daunarubicin, idarubicine or mitoxantrone). This therapeutic regimen is typically followed by administration of high dose cytarabine and/or stem cell transplantation. These treatments have improved outcome in young patients. Progress has also been made in the treatment of acute promyelocytic leukemia, where targeted therapy with all-trans retinoic acid (ATRA) or arsenic trioxide have resulted in excellent survival rates. However, patients over 60, a population which represents the vast majority of AML cases, remain a therapeutic enigma. Although 65-85% of patients initially respond to existing treatments, 65% of such responders undergo relapse, and many patients succumb to the disease. For at least this reason and because the afore-mentioned treatments may have severe side effects, the inventive predictive test can guide use of the treatment that mitigates these litigations. In some embodiments, the present invention improves the likelihood of successful treatment by matching the right patient to the right treatment. Further, there are currently no tests to predict AML patient response to treatment.

The term subject, as used herein unless otherwise defined, is a mammal, *e.g.,* a human, mouse, rat, hamster, guinea pig, dog, cat, horse, cow, goat, sheep, pig, or non-human primate, such as a monkey, chimpanzee, or baboon. The terms "subject" and "patient" are used interchangeably.

*Exemplary Specimens*

In some embodiments, the present invention includes the measurement of a tumor specimen, including biopsy or surgical specimen samples. In some embodiments, the specimen is selected from a frozen tumor tissue specimen, cultured cells, circulating tumor cells, and a formalin-fixed paraffin-embedded tumor tissue specimen (*e.g.* for antibody based BH3 profiling). In some embodiments, the biopsy is a human biopsy. In various embodiments, the biopsy is any one of a frozen tumor tissue specimen, cultured cells, circulating tumor cells, and a formalin-fixed paraffin-embedded tumor tissue specimen (*e.g.* for antibody based BH3 profiling).

In some embodiments, the tumor specimen may be a biopsy sample, such as a frozen tumor tissue (cryosection) specimen. As is known in the art, a cryosection may employ a cryostat, which comprises a microtome inside a freezer. The surgical specimen is placed on a metal tissue disc which is then secured in a chuck and frozen rapidly to about -20°C to about -30°C. The specimen is embedded in a gel like medium consisting of, for example, poly ethylene glycol and polyvinyl alcohol. The frozen tissue is cut frozen with the microtome portion of the cryostat, and the section is optionally picked up on a glass slide and stained.

In some embodiments, the tumor specimen may be a biopsy sample, such as cultured cells. These cells may be processed using the usual cell culture techniques that are known in the art. These cells may be circulating tumor cells.

In some embodiments, the tumor specimen may be a biopsy sample, such as a formalin-fixed paraffin-embedded (FFPE) tumor tissue specimen. As is known in the art, a biopsy specimen may be placed in a container with formalin (a mixture of water and formaldehyde) or some other fluid to preserve it. The tissue sample may be placed into a mold with hot paraffin wax. The wax cools to form a solid block that protects the tissue. This paraffin wax block with the embedded tissue is placed on a microtome, which cuts very thin slices of the tissue.

In certain embodiments, the tumor specimen (or biopsy) contains less than 100 mg of tissue, or in certain embodiments, contains about 50 mg of tissue or less. The tumor specimen (or biopsy) may contain from about 20 mg to about 50 mg of tissue, such as about 35 mg of tissue.

The tissue may be obtained, for example, as one or more (*e.g.,* 1, 2, 3, 4, or 5) needle biopsies (*e.g.,* using a 14-gauge needle or other suitable size). In some embodiments, the biopsy is a fine-needle aspiration in which a long, thin needle is inserted into a suspicious area and a syringe is used to draw out fluid and cells for analysis. In some embodiments, the biopsy is a core needle biopsy in which a large needle with a cutting tip is used during core needle biopsy to draw a column of tissue out of a suspicious area. In some embodiments, the biopsy is a vacuum-assisted biopsy in which a suction device increases the amount of fluid and cells that is extracted through the needle. In some embodiments, the biopsy is an image-guided biopsy in which a needle biopsy is combined with an imaging procedure, such as, for example, X ray, computerized tomography (CT), magnetic resonance imaging (MRI) or ultrasound. In other embodiments, the sample may be obtained via a device such as the MAMMOTOME® biopsy system, which is a laser guided, vacuum-assisted biopsy system for breast biopsy.

In certain embodiments, the specimen is a human tumor-derived cell line. In certain embodiments, the specimen is a cancer stem cell. In other embodiments, the specimen is derived from the biopsy of a solid tumor, such as, for example, a biopsy of a colorectal, breast, prostate, lung, pancreatic, renal, or ovarian primary tumor.

In certain embodiments, the specimen is of epithelial origin. In some embodiments, the epithelial specimen is enriched by selection from a biopsy sample with an anti-epithelial cell adhesion molecule (EpCAM) or other epithelial cell binding antibody bound to solid matrix or bead.

In certain embodiments, the specimen is of mesenchymal origin. In some embodiments, the mesenchymal specimen is enriched by selection from a biopsy sample with a neural cell adhesion molecule (N-CAM) or neuropilin or other mesenchymal cell binding antibody bound to a solid matrix or bead.

In certain embodiments, the specimen is derived from the biopsy of a non-solid tumor, such as, for example, any of the cancer described herein. In a specific embodiment, the specimen is a multiple myeloma cell that is enriched by selection from a biopsy sample with an anti-CD138 antibody bound to a solid matrix or bead. In a specific embodiment, the specimen is an acute myelogenous leukemia cell that is enriched by binding to a CD45-directed antibody.

In some embodiments, the specimen is derived from a circulating tumor cell.

*BH3 Profiling*

The invention comprises BH3 profiling. In various embodiments, the invention comprises BH3 profiling in which at least two, or three, or four, or five, or six, or seven, or eight, or nine, or ten BH3 peptides are evaluated at once. In some embodiments, the present methods comprise a multipeptide analysis, as opposed to an evaluation of a single BH3 peptide. In some embodiments, a panel of BH3 peptides is screened on a single patient specimen.

In some embodiments, the BH3 profiling comprises use of a peptide, wherein the peptide is one or more of BIM, BIM2A, BAD, BID, HRK, PUMA, NOXA, BMF, BIK, and PUMA2A. In some embodiments, the BH3 profiling comprises use of an antibody directed against one of more of BIM, BIM2A, BAD, BID, HRK, PUMA, NOXA, BMF, BIK, and PUMA2A and naturally-occurring heterodimers formed between two Bcl-2 proteins, *e.g.* a first Bcl-2 protein (*e.g.,* Bim, Bid, Bad, Puma, Noxa, Bak, Hrk, Bax, or Mule) and a second Bcl-2 protein (*e.g.*, Mcl-1, Bcl-2, Bcl-XL, Bfl-1 or Bcl-w) as described in U.S. Patent No. 8,168,755. In some embodiments the BH3 profiling comprises use of a stapled peptide (*e.g.* a peptide generated through the synthetic enhancement of a 3-D alpha-helix protein segment with hydrocarbon bonds to make proteins more rigid and able to penetrate cells), as described in, for example, Verdine, et al. "Stapled Peptides for Intracellular Drug Targets" Methods in Enzymology, Volume 503 (Chap. 1).

In one embodiment, the peptide is used at a concentration of about 0.1 to about 200 $\mu$M. In some embodiments,

about 0.1 to about 150, or about 0.1 to about 100, or about 0.1 to about 50, or about 0.1 to about 10, or about 0.1 to about 5, about 1 to about 150, or about 1 to about 100, about 1 to about 50, about 1 to about 10, about 1 to about 5 $\mu$M, or about 10 to about 100 $\mu$M of the peptide is used. In some embodiments, a concentration of about 0.1, or about 0.5, or about 1.0, or about 5, or about 10, or about 50, or about 100, or about 150, or about 200 $\mu$M of the peptide is used. In one embodiment, the BH3 profiling comprises permeabilizing a specimen.

BH3 profiling and reagents useful for such a method is described in U.S. Patent Nos. 7,868,133; 8,221,966; and 8,168,755 and US Patent Publication No. 2011/0130309.

Briefly, without wishing to be bound by theory, as a result of aberrant phenotypes, cancer cells develop blocks in apoptosis pathways. These blocks make cancer cells both resistant to some therapies, and, surprisingly, make some cancer cells sensitive to other therapies. The concept of "oncogene addiction" describes the phenomena of the acquired dependence of cancer cells on, or addiction to, particular proteins for survival. BH3 profiling determines if such a dependence on certain apoptosis regulating proteins occurs in given cancer cells, and identifies the dependent protein. Cancer cells can be, but are not always, pre-set to undergo apoptosis and this is a function of these cells being dependent on any, or all of the anti-apoptotic Bcl-2 family proteins for their otherwise unintended survival. This provides insight into the likelihood of a cancer cell to respond to treatment.

Cancer cells, without wishing to be bound by theory, exhibit abnormalities, such as DNA damage, genetic instability, abnormal growth factor signaling, and abnormal or missing matrix interactions, any of which should typically induce apoptosis through the intrinsic (mitochondrial) apoptosis pathway. However, rather than respond to these apoptosis signals cancer cells survive. Often, in doing so, these cells become highly dependent on selected blocks to chronic apoptosis signals. This adaptation provides a survival mechanism for the cancer cells; however, these adaptations can also make cancer cells susceptible to particular apoptosis inducing therapies. A crucial event that commits a cell to die by intrinsic apoptosis is the permeabilization of the mitochondrial outer membrane (MOMP) and the release of molecules that activate the effector caspases. In many cases, MOMP is the point of no return in the intrinsic apoptosis pathway. The Bcl-2 family proteins are the key regulators of MOMP, and their activity is linked to the onset of lymphoid and several solid tumor cancers and is believed in many cancers to be the key mediator of resistance to chemotherapy.

Bcl-2 proteins are regulated by distinct protein-protein interactions between pro-survival (anti-apoptotic) and pro-apoptotic members. These interactions occur primarily through BH3 (Bcl-2 homology domain-3) mediated binding. Apoptosis-initiating signaling occurs for the most part upstream of the mitochondria and causes the translocation of short, BH3-only, Bcl-2 family members to the mitochondria where they either activate or sensitize MOMP. The activator BH3 only proteins, Bim and Bid, bind to and directly activate the effector, pro-apoptotic proteins Bax and Bak, and also bind to and inhibit the anti-apoptotic Bcl-2 family proteins, Bcl-2, Mcl-1, Bfl-1, Bcl-w and Bcl-xL. The sensitizer BH3 proteins, Bad, Bik, Noxa, Hrk, Bmf and Puma, bind only to the anti-apoptotic Bcl-2 family proteins, Bcl-2, Mcl-1, Bfl-1, Bcl-w and Bcl-xL, blocking their anti-apoptotic functions. Without wishing to be bound by theory, each sensitizer protein has a unique specificity profile. For example, Noxa (A and B) bind with high affinity to Mcl-1, Bad binds to Bcl-xL and Bcl-2 but only weakly to Mcl-1, and Puma binds well to all three targets. An anti-apoptotic function of these proteins is the sequestering of the activator BH3 protein Bim and Bid. Displacement of these activators by sensitizer peptides results in Bax/Bak-mediated apoptotic commitment. These interactions can have various outcomes, including, without limitation, homeostasis, cell death, sensitization to apoptosis, and blockade of apoptosis.

A defining feature of cancer cells in which apoptotic signaling is blocked is an accumulation of the BH3 only activator proteins at the mitochondrial surface, a result of these proteins being sequestered by the anti-apoptotic proteins. This accumulation and proximity to their effector target proteins accounts for increased sensitivity to antagonism of Bcl-2 family proteins in the "BH3 primed" state.

In some embodiments, a cell yielding a high apoptotic response to Noxa (A or B) is Mcl-1 primed, while a high response to the peptide Bad indicates that Bcl-xL or Bcl-2 provides the apoptotic block. In some embodiments, Puma reflects pan-Bcl-2 family priming. In this way, cells that are dependent on either Mcl-1 or Bcl-xL, on both proteins, or on several Bcl-2 family members are readily distinguished so that appropriate treatment may be tailored accordingly. The distinctions in mitochondrial response to these peptides guides the use of therapies that are known to work through pathways that funnel into either Mcl-1 or Bcl-xL affected intrinsic signaling. The use of a Bcl-2 inhibiting or a Mcl-1 inhibiting compound may be indicated in such cases. In some embodiments, the present methods also indicate or contraindicate therapies that target entities upstream of Mcl-1 or Bcl-xL.

BH3 profiling assay identifies when a cancer cell is in the primed state, as well as in which configuration the priming has occurred and this has predictive value.

*Exemplary Clinical Factors and Additional Biomarkers*

The invention comprises the evaluation of clinical factors. In some embodiments, a clinical factor that provides patient response information in combination with a BH3 profiling study may not be linked to apoptosis. In some embodiments, a clinical factor is non-apoptosis affecting.

In one embodiment, the clinical factor is age. In one embodiment, the patient age profile is classified as over about 10, or over about 20, or over about 30, or over about 40, or over about 50, or over about 60, or over about 70, or over about 80 years old.

In one embodiment, the clinical factor is cytogenetic status. In some cancers, such as Wilms tumor and retinoblastoma, for example, gene deletions or inactivations are responsible for initiating cancer progression, as chromosomal regions associated with tumor suppressors are commonly deleted or mutated. For example, deletions, inversions, and translocations are commonly detected in chromosome region 9p21 in gliomas, non-small-cell lung cancers, leukemias, and melanomas. Without wishing to be bound by theory, these chromosomal changes may inactivate the tumor suppressor cyclin-dependent kinase inhibitor 2A. Along with these deletions of specific genes, large portions of chromosomes can also be lost. For instance, chromosomes 1p and 16q are commonly lost in solid tumor cells. Gene duplications and increases in gene copy numbers can also contribute to cancer and can be detected with transcriptional analysis or copy number variation arrays. For example, the chromosomal region 12q13-q14 is amplified in many sarcomas. This chromosomal region encodes a binding protein called MDM2, which is known to bind to a tumor suppressor called p53. When MDM2 is amplified, it prevents p53 from regulating cell growth, which can result in tumor formation. Further, certain breast cancers are associated with overexpression and increases in copy number of the *ERBB2* gene, which codes for human epidermal growth factor receptor 2. Also, gains in chromosomal number, such as chromosomes 1q and 3q, are also associated with increased cancer risk.

Cytogenetic status can be measured in a variety of manners known in the art. For example, FISH, traditional karyotyping, and virtual karyotyping (*e.g.* comparative genomic hybridization arrays, CGH and single nucleotide polymorphism arrays) may be used. For example, FISH may be used to assess chromosome rearrangement at specific loci and these phenomenon are associated with disease risk status. In some embodiments, the cytogentic status is favorable, intermediate, or unfavorable.

In one embodiment, the clinical factor is performance. Performance status can be quantified using any system and methods for scoring a patient's performance status are known in the art. The measure is often used to determine whether a patient can receive chemotherapy, adjustment of dose adjustment, and to determine intensity of palliative care. There are various scoring systems, including the Karnofsky score and the Zubrod score. Parallel scoring systems include the Global Assessment of Functioning (GAF) score, which has been incorporated as the fifth axis of the Diagnostic and Statistical Manual (DSM) of psychiatry. Higher performance status (*e.g.*, at least 80%, or at least 70% using the Karnofsky scoring system) may indicate treatment to prevent progression of the disease state, and enhance the patient's ability to accept chemotherapy and/or radiation treatment. For example, in these embodiments, the patient is ambulatory and capable of self care. In other embodiments, the evaluation is indicative of a patient with a low performance status (*e.g.*, less than 50%, less than 30%, or less than 20% using the Karnofsky scoring system), so as to allow conventional radiotherapy and/or chemotherapy to be tolerated. In these embodiments, the patient is largely confined to bed or chair and is disabled even for self-care.

The Karnofsky score runs from 100 to 0, where 100 is "perfect" health and 0 is death. The score may be employed at intervals of 10, where: 100% is normal, no complaints, no signs of disease; 90% is capable of normal activity, few symptoms or signs of disease, 80% is normal activity with some difficulty, some symptoms or signs; 70% is caring for self, not capable of normal activity or work; 60% is requiring some help, can take care of most personal requirements; 50% requires help often, requires frequent medical care; 40% is disabled, requires special care and help; 30% is severely disabled, hospital admission indicated but no risk of death; 20% is very ill, urgently requiring admission, requires supportive measures or treatment; and 10% is moribund, rapidly progressive fatal disease processes.

The Zubrod scoring system for performance status includes: 0, fully active, able to carry on all pre-disease performance without restriction; 1, restricted in physically strenuous activity but ambulatory and able to carry out work of a light or sedentary nature, *e.g.*, light house work, office work; 2, ambulatory and capable of all self-care but unable to carry out any work activities, up and about more than 50% of waking hours; 3, capable of only limited self-care, confined to bed or chair more than 50% of waking hours; 4, completely disabled, cannot carry on any self-care, totally confined to bed or chair; 5, dead.

In one embodiment, the clinical factor is histological subclass. In some embodiments, histological samples of tumors are graded according to Elston & Ellis, Histopathology, 1991, 19:403-10.

In one embodiment, the clinical factor is gender. In one embodiment, the gender is male. In another embodiment the gender is female.

In one embodiment, the clinical factor is disease stage. By way of non-limiting example, using the overall stage grouping, Stage I cancers are localized to one part of the body; Stage II cancers are locally advanced, as are Stage III cancers. Whether a cancer is designated as Stage II or Stage III can depend on the specific type of cancer. In one non-limiting example, Hodgkin's disease, Stage II indicates affected lymph nodes on only one side of the diaphragm, whereas Stage III indicates affected lymph nodes above and below the diaphragm. The specific criteria for Stages II and III therefore differ according to diagnosis. Stage IV cancers have often metastasized, or spread to other organs or throughout the body.

In some embodiments, the clinical factor is the French-American-British (FAB) classification system for hematologic diseases (*e.g.* indicating the presence of dysmyelopoiesis and the quantification of myeloblasts and erythroblasts). In one embodiment, the FAB for acute lymphoblastic leukemias is L1-L3, or for acute myeloid leukemias is M0-M7.

In another embodiment, the clinical response is about 1, about 2, about 3, or about 5 year progression/event-free survival.

A variety of clinical factors have been identified, such as age profile and performance status. A number of static measurements of diagnosis have also been utilized, such as cytogenetics and molecular events including, without limitation, mutations in the genes MLL, AML/ETO, Flt3-ITD, NPM1 (NPMc+), CEBPα, IDH1, IDH2, RUNX1, ras, and WT1 and in the epigenetic modifying genes TET2 and ASXL, as well as changes in the cell signaling protein profile.

The term "about" when used in connection with a referenced numeric indication means the referenced numeric indication plus or minus up to 10% of that referenced numeric indication. For example, the language "about 50" covers the range of 45 to 55.

As used herein, the word "include," and its variants, is intended to be non-limiting, such that recitation of items in a list is not to the exclusion of other like items that may also be useful in the materials, compositions, devices, and methods of this technology. Similarly, the terms "can" and "may" and their variants are intended to be non-limiting, such that recitation that an embodiment can or may comprise certain elements or features does not exclude other embodiments of the present technology that do not contain those elements or features. Although the open-ended term "comprising," as a synonym of terms such as including, containing, or having, is used herein to describe and claim the invention, the present technology, or embodiments thereof, may alternatively be described using more limiting terms such as "consisting of" or "consisting essentially of" the recited ingredients.

Unless defined otherwise, all technical and scientific terms herein have the same meaning as commonly understood by one of ordinary skill in the art to which this invention belongs.

This invention is further illustrated by the following non-limiting examples.

## EXAMPLES

### Example 1: Cell-Based Studies in Therapeutic Development

*Methodology*: For test cells, $7.5 \times 10^3$ cells per well were suspended in reaction buffer (300 mM Trehalose, HEPES-KOH pH 7.4. 80 mM KCl, 1 mM EGTA, 1 mM EDTA, 0.1% BSA and 5 mM Succinate). The cells were permeabilized with digitonin and loaded with the cationic dye JC-1, and β-mercaptoethanol. The cells were then aliquoted into the wells of a 384-well microtiter plate and incubated with one of the BH3 domain peptides: Bim, Bid, Bad, NoxaA, Bim2A, Puma, Bmf, Hrk and Bik. Peptides used in this assay were synthesized and were >95% pure, as determined by HPLC. Peptide identity was confirmed by mass spectrometry. A DMSO vehicle control was included as a negative control. Full mitochondrial membrane depolarization was measured by treating the cells with 1 mM FCCP (p-trifluoromethoxy carbonyl cyanide phenyl hydrazone), and this sample served as an assay standard and positive control. Peptide (and FCCP) addition resulted in a decrease in membrane potential in suitably primed cells, which is measured as a decrease in JC-1 fluorescence on a Tecan Genios plate reader using an excitation of 545 nM and an emission of 590 nm. The kinetics of this process varied for each peptide, and endpoints were achieved and recorded from kinetic traces over a 180 minute time course. Fluorescence decrease for each peptide was normalized to the FCCP response, and reported as percent loss of membrane potential, %DYm. Each experiment was performed in triplicate for each of the cell lines.

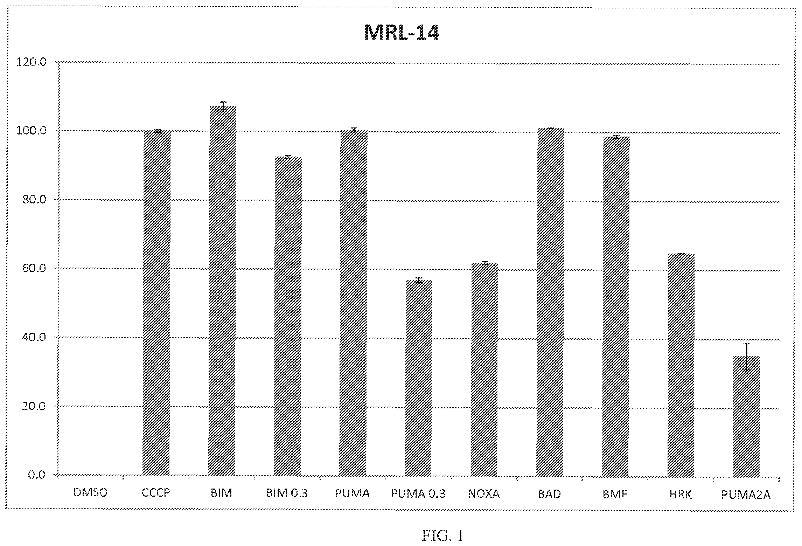

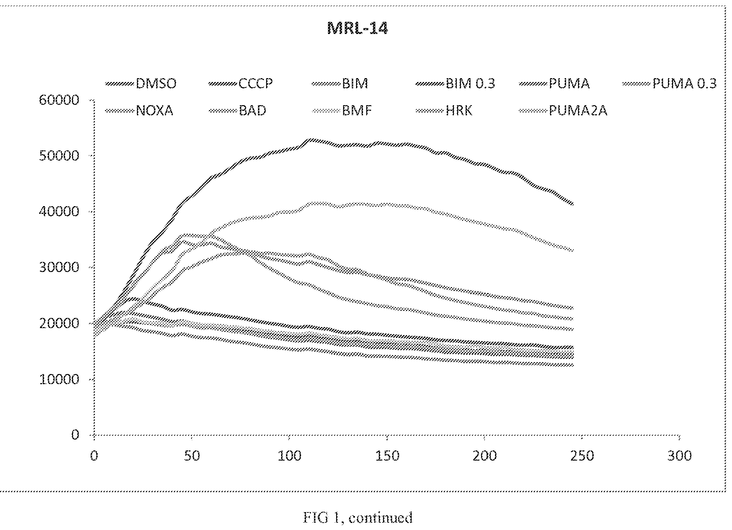

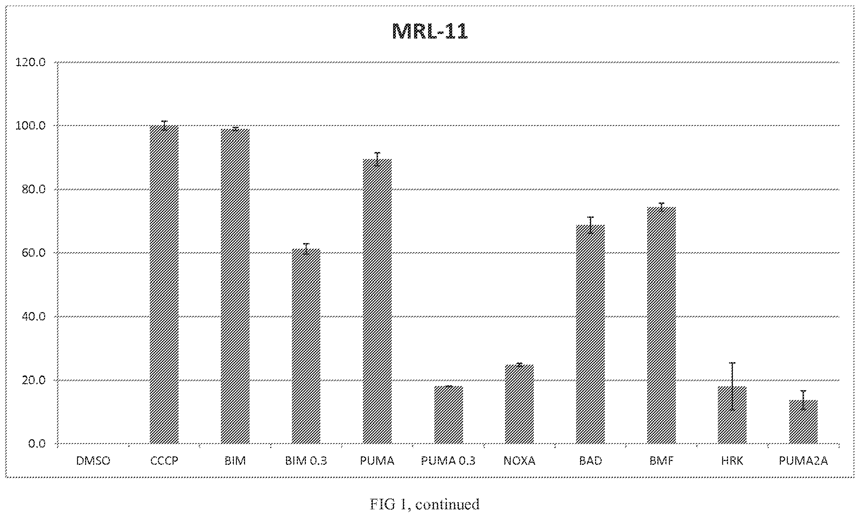

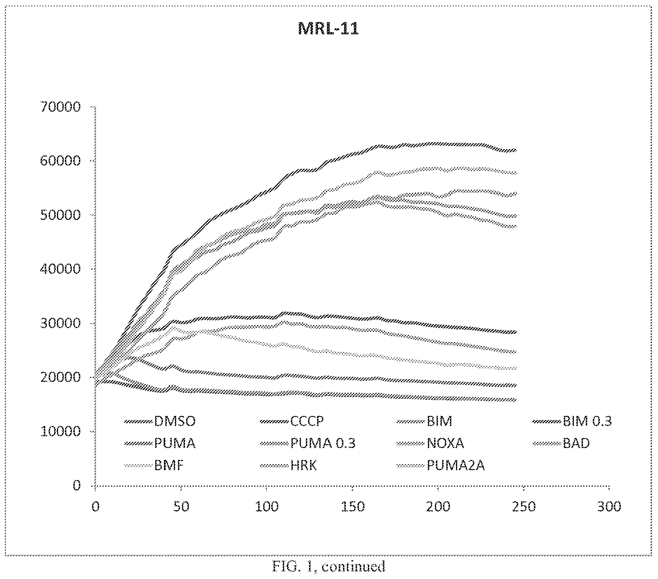

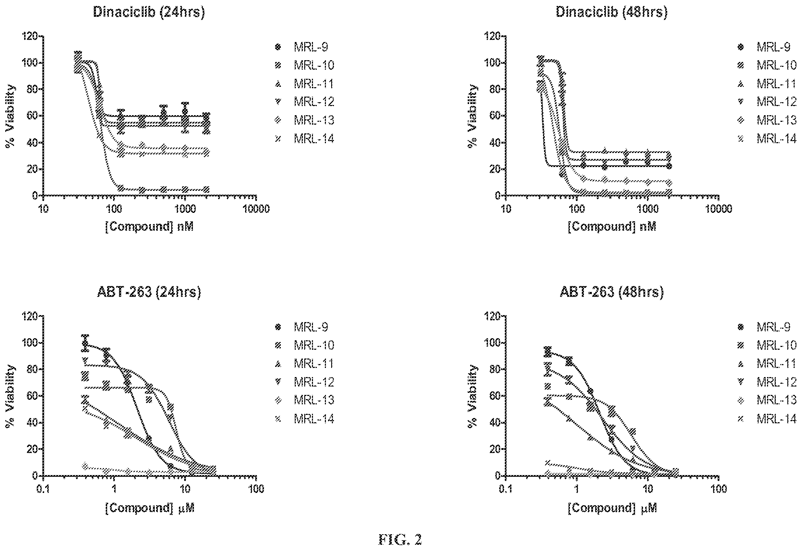

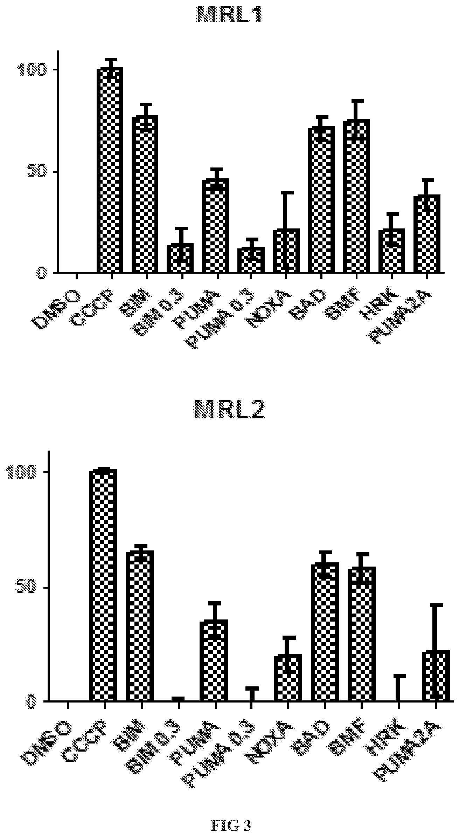

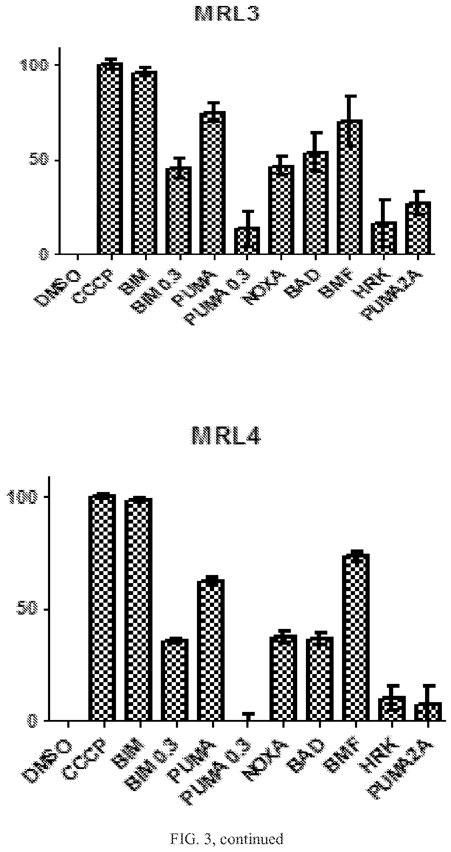

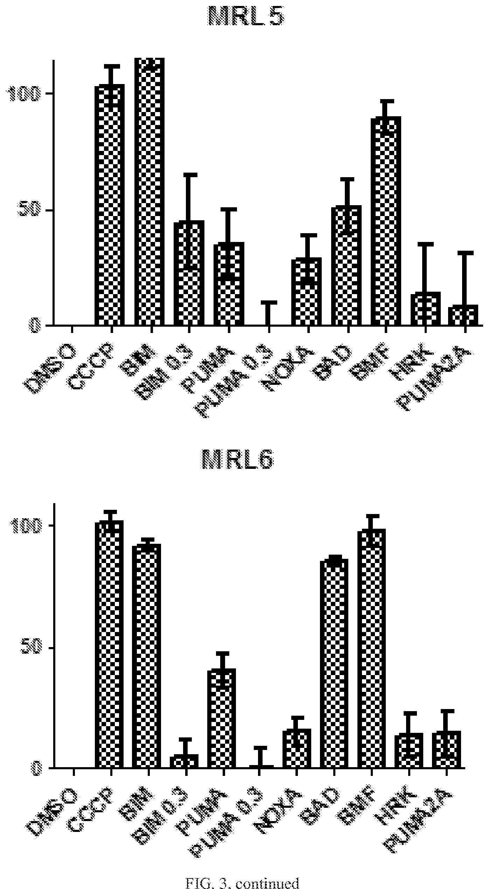

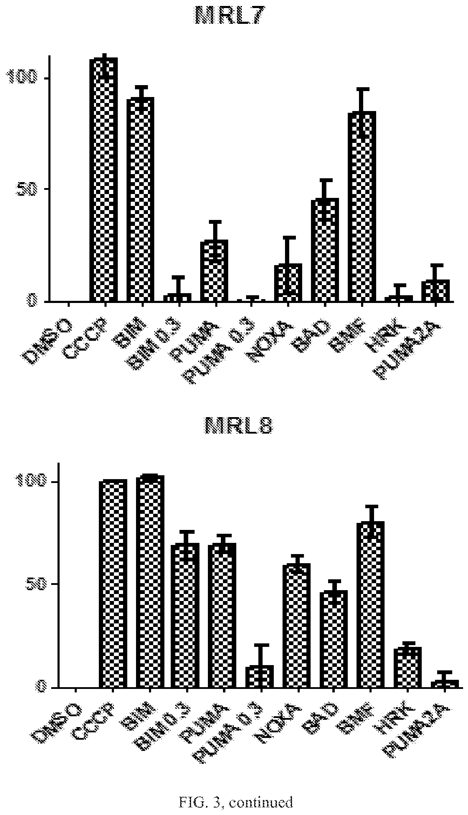

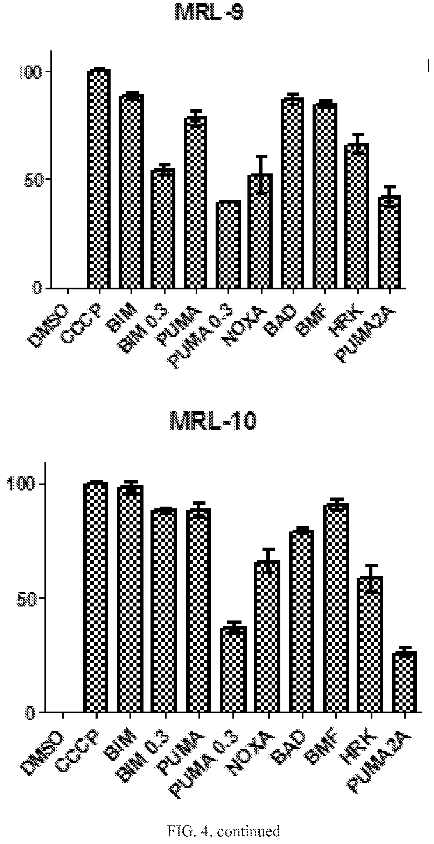

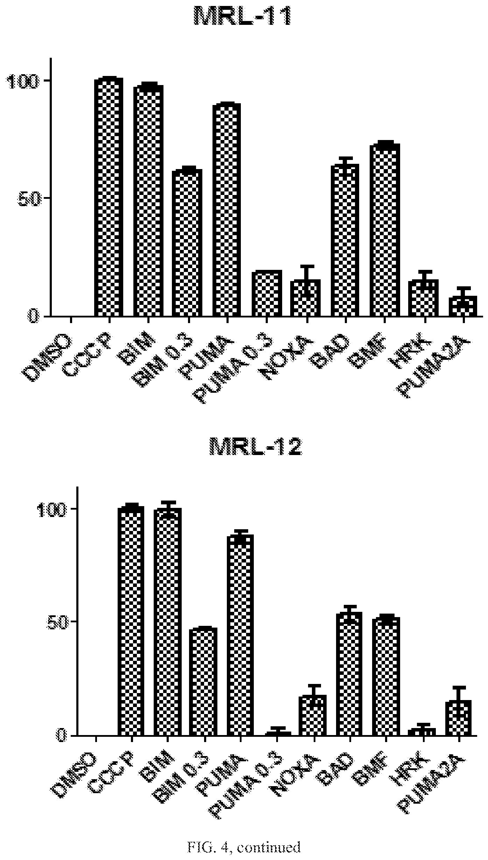

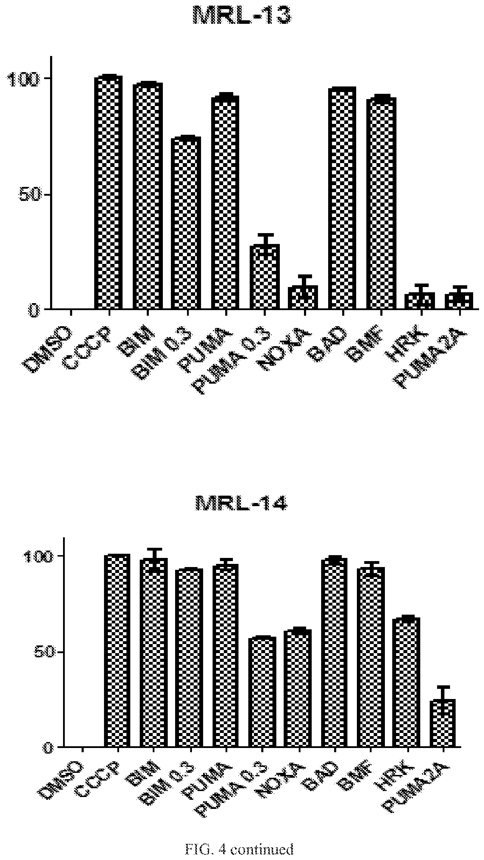

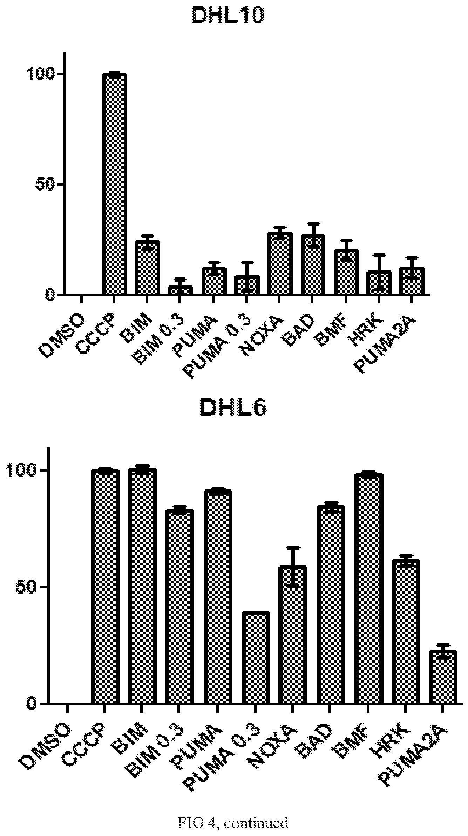

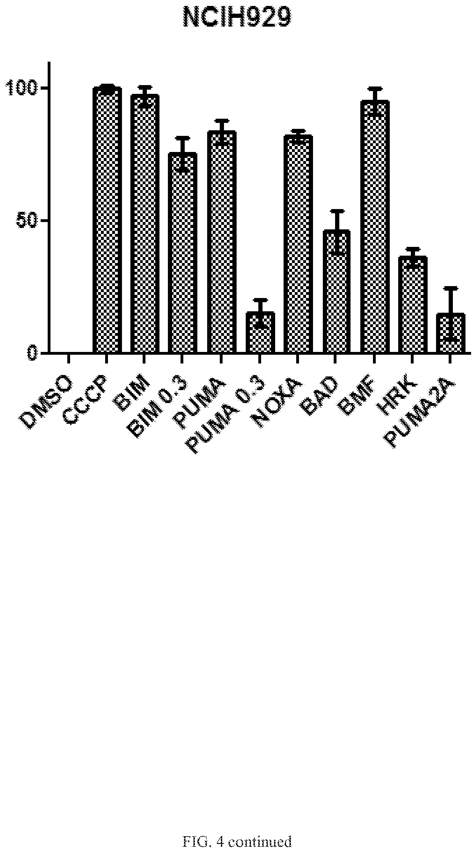

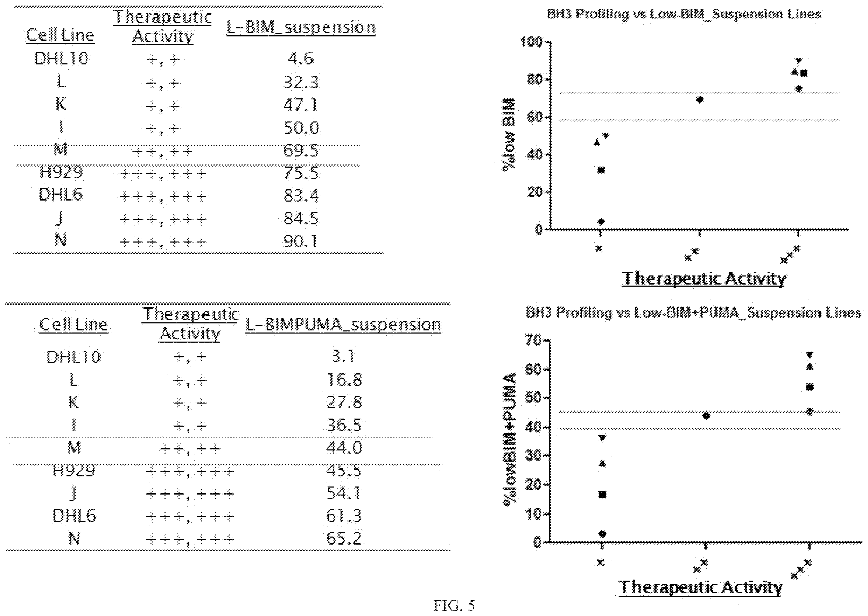

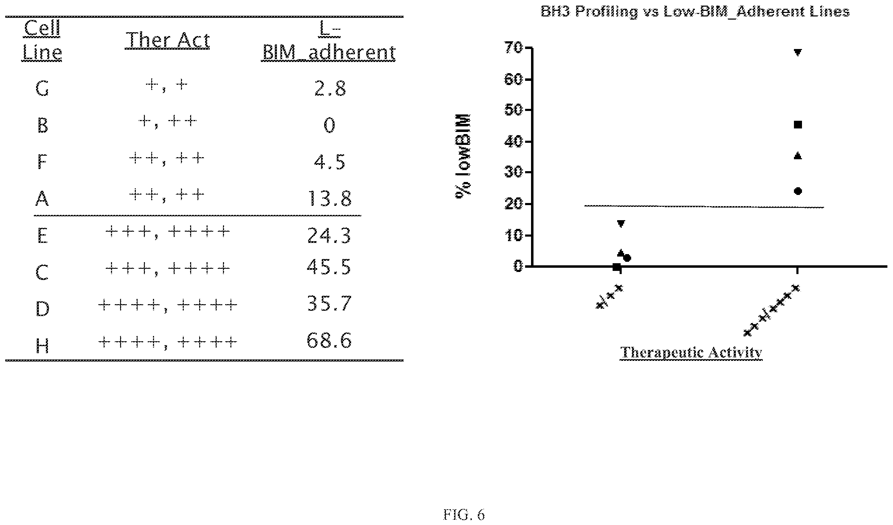

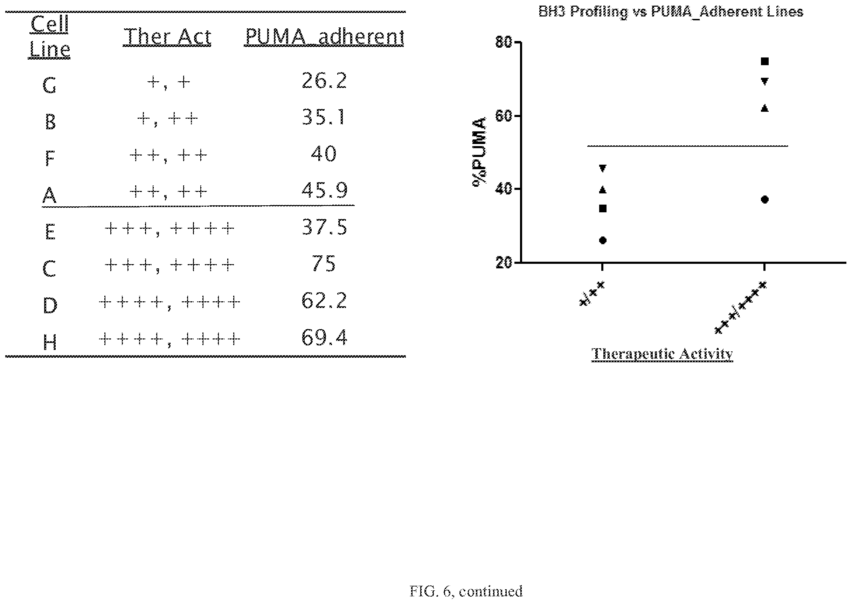

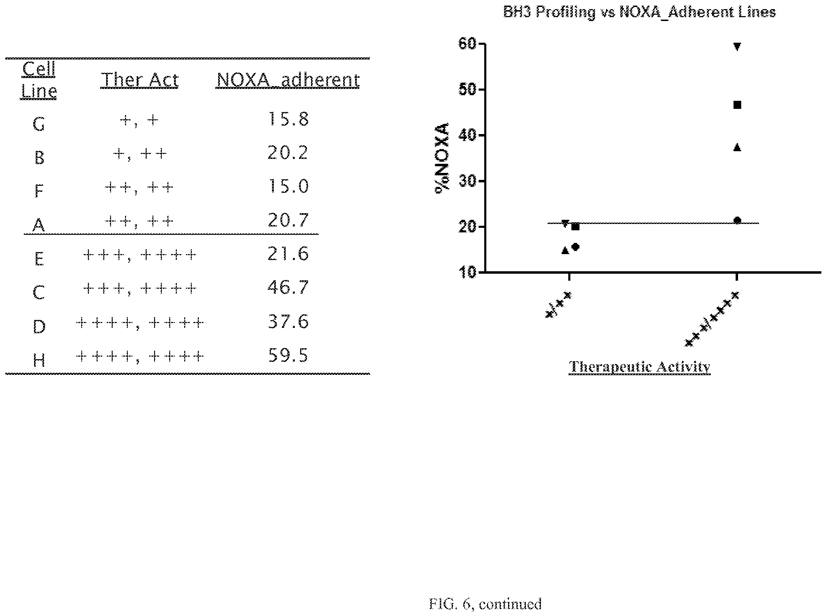

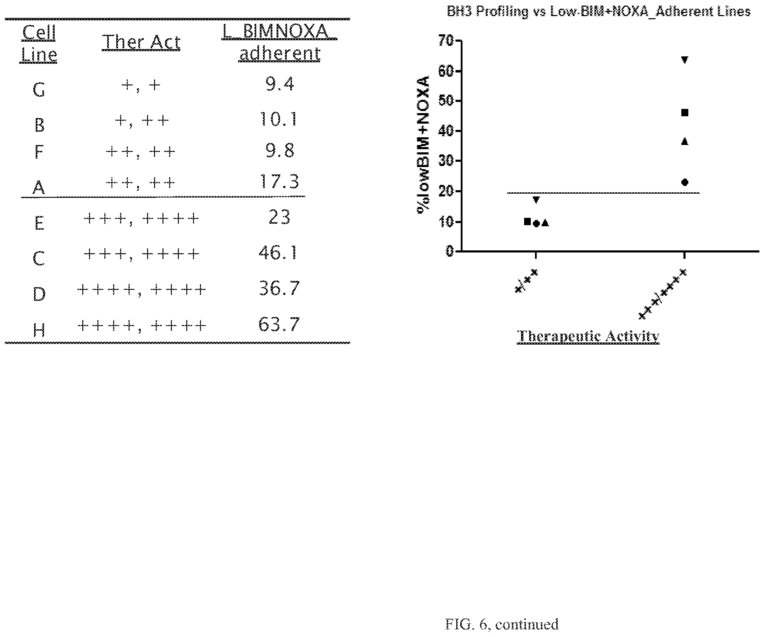

*Cyclin-dependent kinase inhibitor development:* Correlation of BH3 profiling with cell lines binned by therapeutic inhibitor activity indicates that pro-apoptotic peptides discriminate has been utilized on cell lines to discriminate response to developmental therapeutics affecting the cell cycle. Representative BH3 profiling data from such cell lines is indicated in **FIG. 1** and cell cycle modulating agents therapeutic efficacy on said cell lines is indicated in **FIG. 2**. The complete BH3 profiles for a panel of 8 adherent lines derived from solid tumor malignancies is shown in **FIG. 3** while the BH3 profiles for all peptides tested against suspension lines derived from non-solid tumors is indicated in **FIG. 4**. Therapeutic efficacy for cyclin dependent kinase inhibitor in both adherent and suspension lines as well as the quantified BH3 profiling metrics is summarized in **Table 1**. Specifically, percent response to low BIM peptide correlates with therapeutic activity in both suspension as well as adherent lines (**FIGs. 5** and **6**). Percent response to NOXA and PUMA peptides also trended toward discrimination, especially when combined with BIM (NOXA+BIM_adherent; PUMA+BIM_suspension).

Cell cycle modulators are inherently designed to disrupt the cell cycle and thus serve as predominantly cytostatic agents. BH3 profiling, on the other hand, is designed to measure the propensity of a cell to undergo pro-apoptotic cues. Accordingly, the data described in this Example show, *inter alia,* the unexpected diagnostic use of the present BH3 profiling approach to be predicative of therapeutic efficacy of agents not mechanistically linked to pro-apoptotic cues.

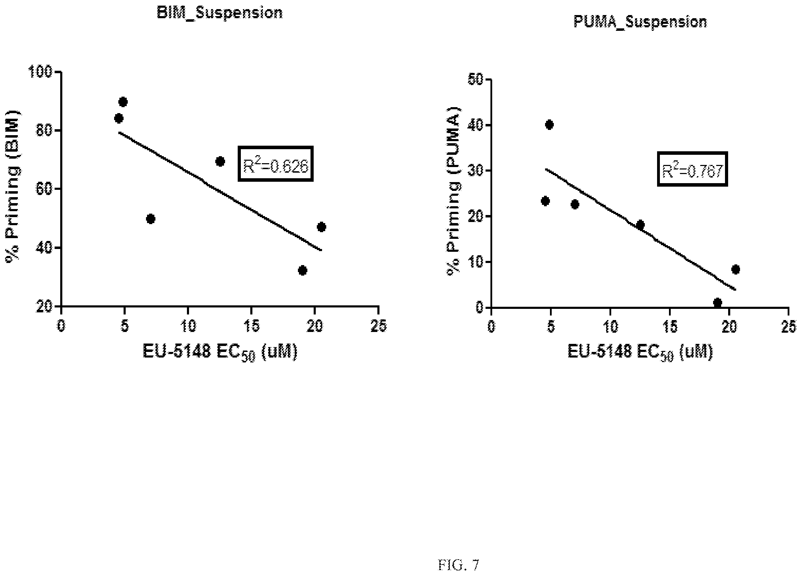

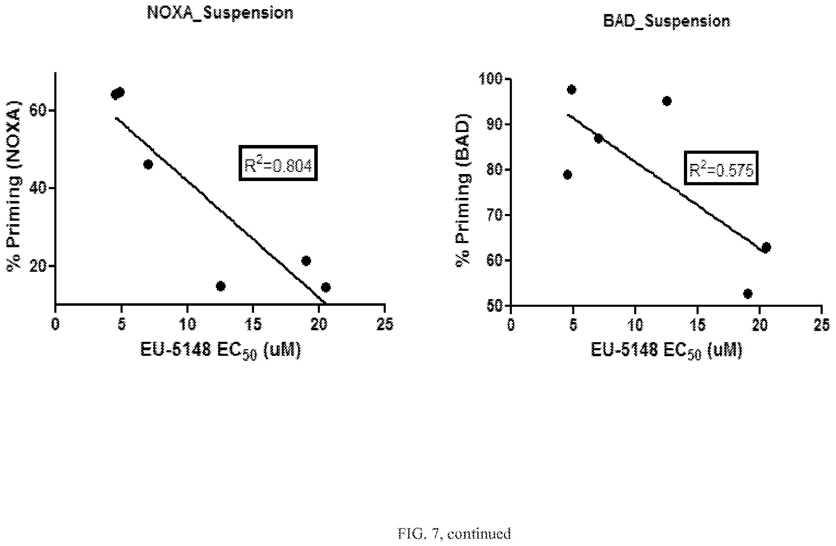

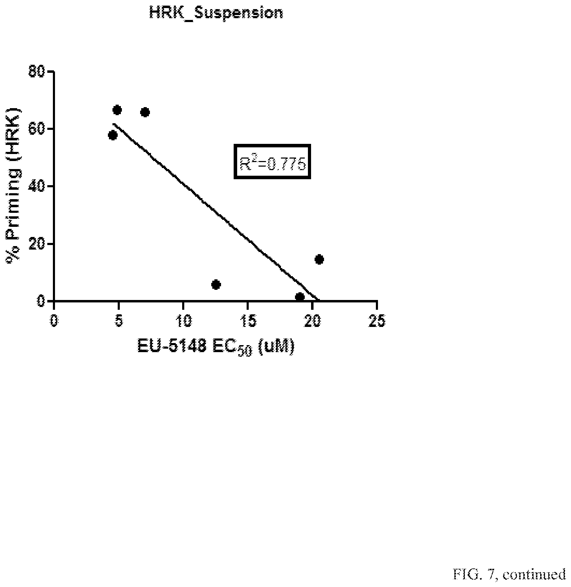

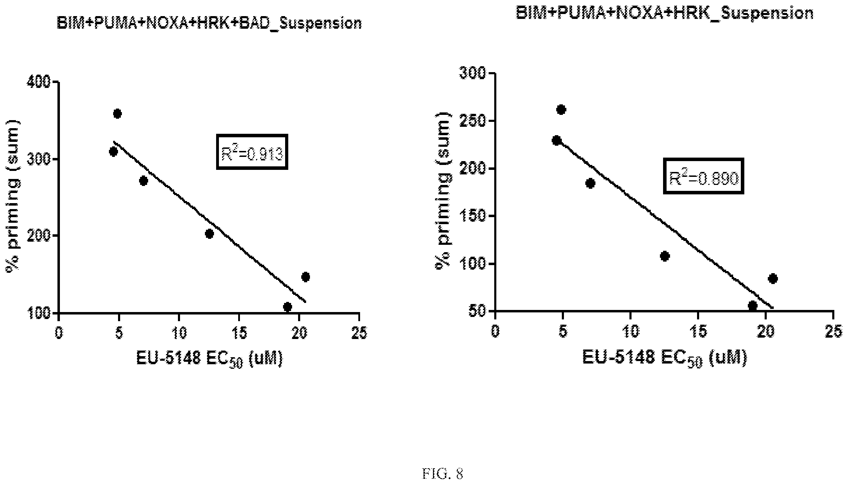

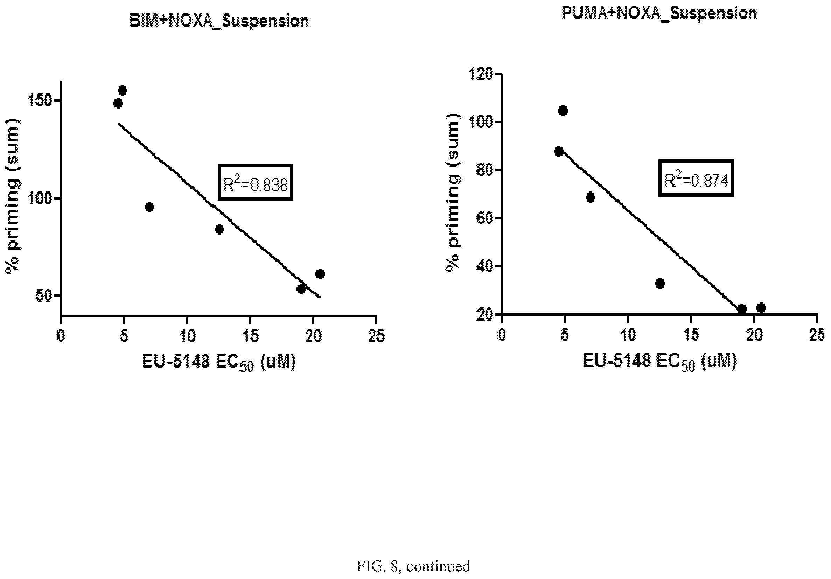

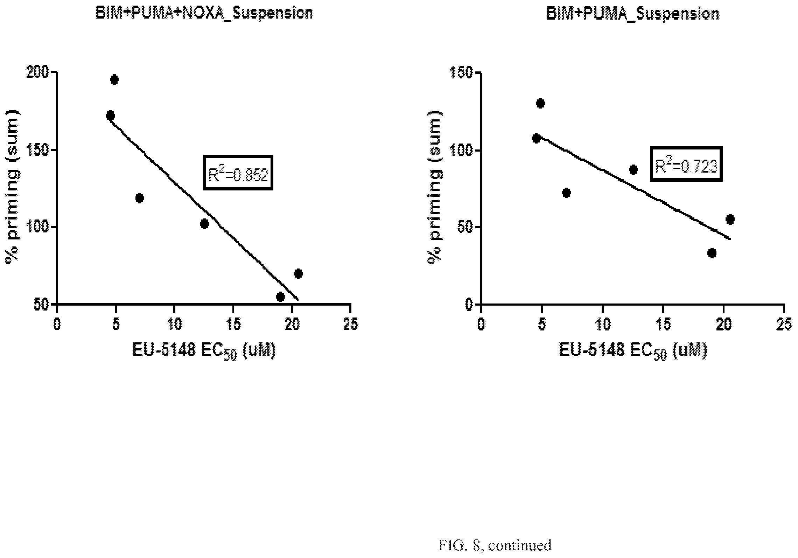

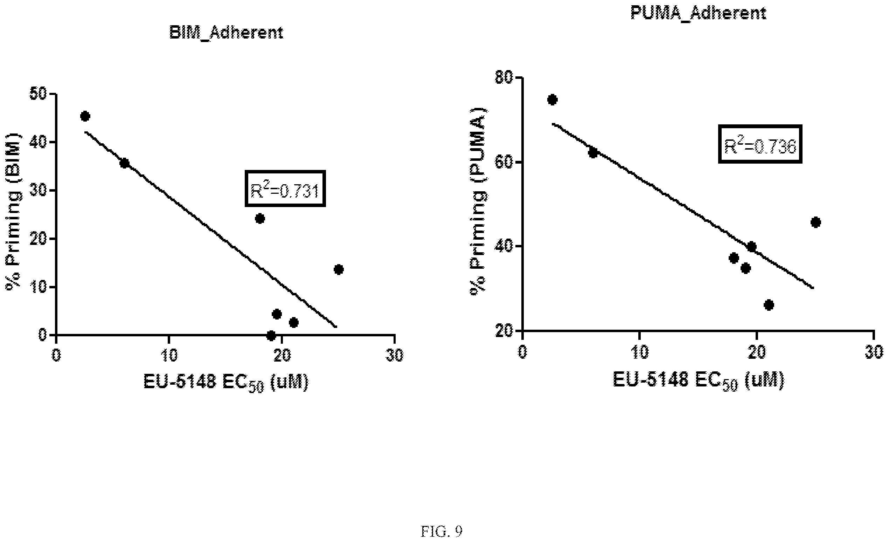

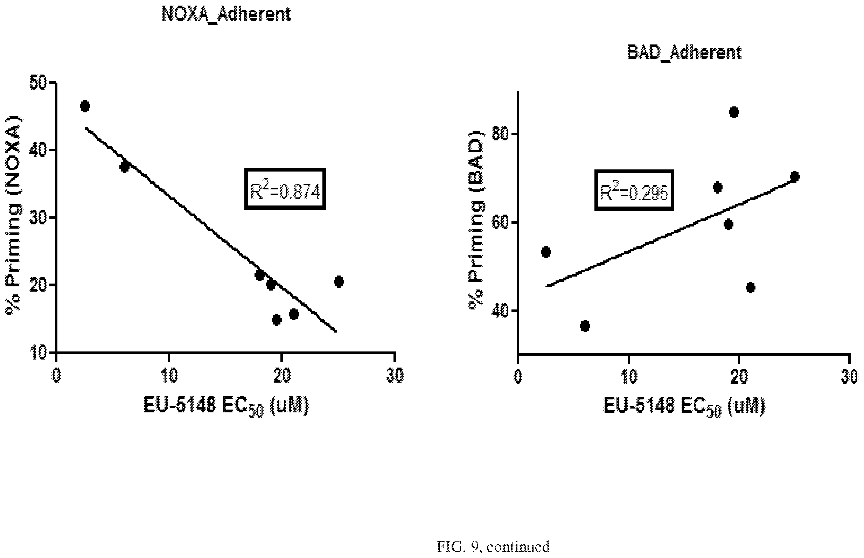

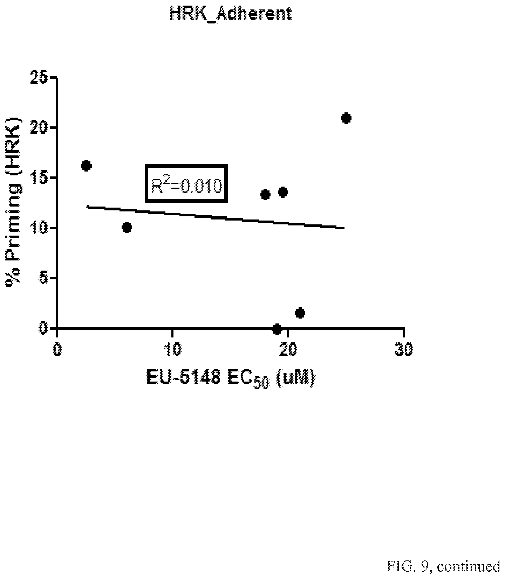

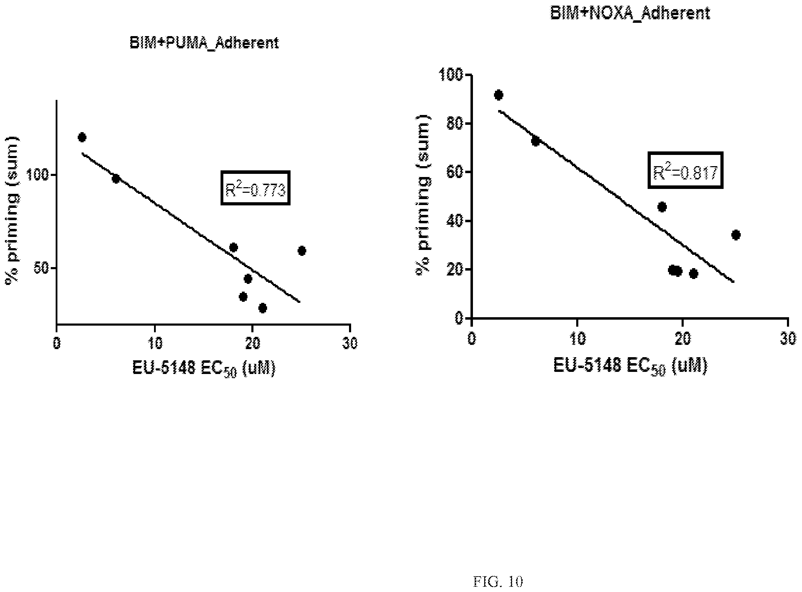

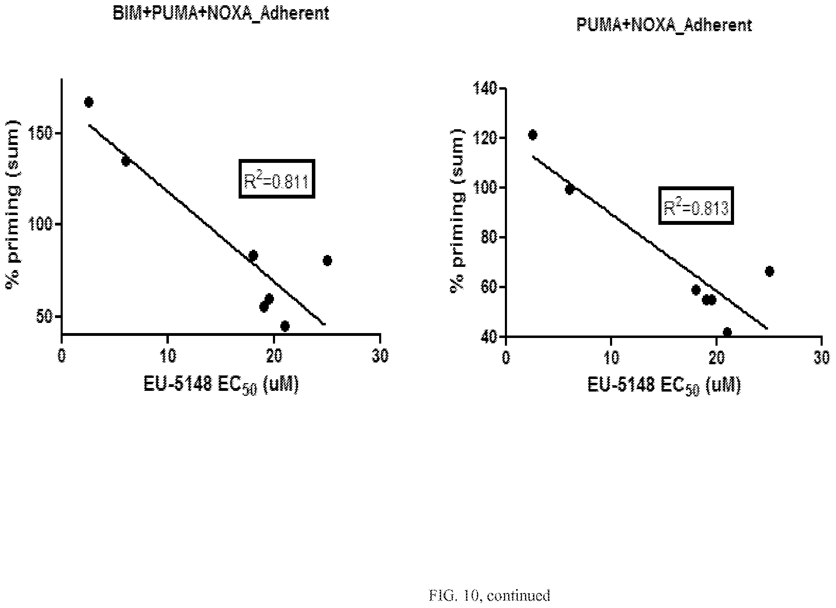

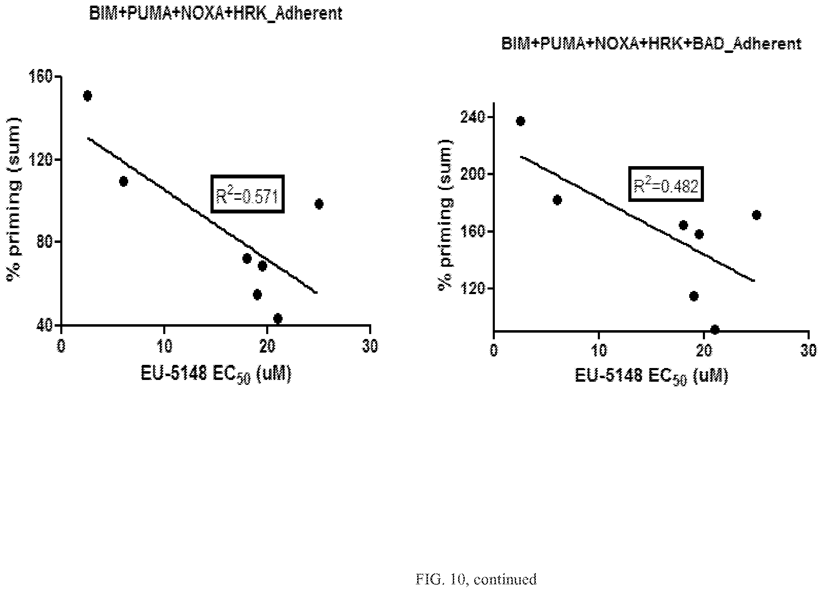

*BH3 mimetics (MCL-1 inhibitor development)*: Correlation of BH3 profiling with cell lines binned by therapeutic inhibitor activity indicates that pro-apoptotic peptides discriminate efficacy in both suspension (**FIG. 7**) and adherent cells (**FIG. 9**). Specifically, percent response to low BIM, PUMA, NOXA, BAD and HRK peptides all individually correlate with therapeutic activity in suspension lines. Multimarker algorithms that are correlated with therapeutic efficacy in suspension lines include BIM+PUMA, BIM+NOXA, PUMA+NOXA, BIM+PUMA+NOXA, BIM+PUMA+NOXA+HRK, and BIM+PUMA+NOXA+HRK+BAD (**FIG. 8**). In adherent lines from solid tumors, BIM, PUMA and NOXA are each individually correlated with MCL-1 inhibitor efficacy, while the multimarker algorithms that show correlation are the same ones as previously mentioned for correlation in suspension lines (BIM+PUMA, BIM+NOXA, PUMA+NOXA, BIM+PUMA+NOXA, BIM+PUMA+NOXA+HRK, and BIM+PUMA+NOXA+HRK+BAD) (**FIG. 10**).

This data shows, *inter alia,* that multimarker approaches may have added information over individual markers when predicting therapeutic efficacy. Such findings are unexpected in light of MCL-1 principally being modulated by Noxa specifically and PUMA (also binding to BCL2 and BCLxl) non-specifically. The weighting conferred by inclusion of multiple markers within such an approach provides a correlation to therapeutic efficacy than an MCL-1 inhibitor individiually. Data for therapeutic efficacy and BH3 profiling individual and multi-marker algorithms is provided in **Table 2.**

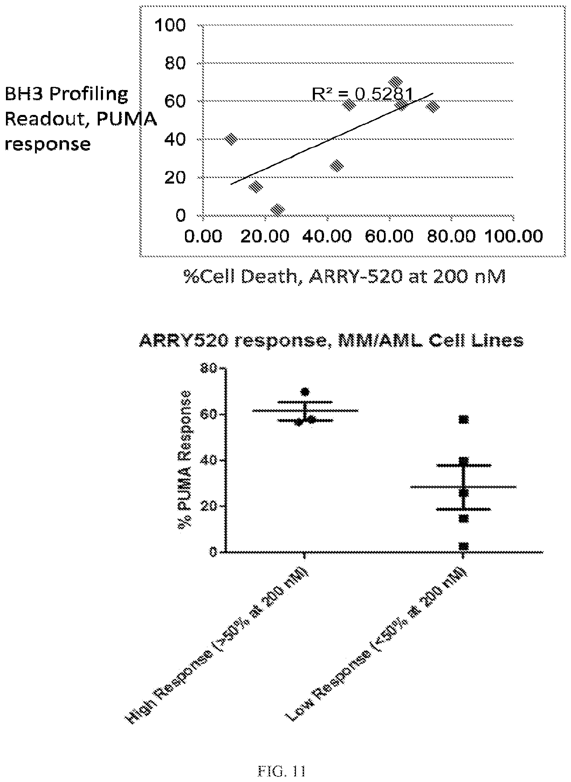

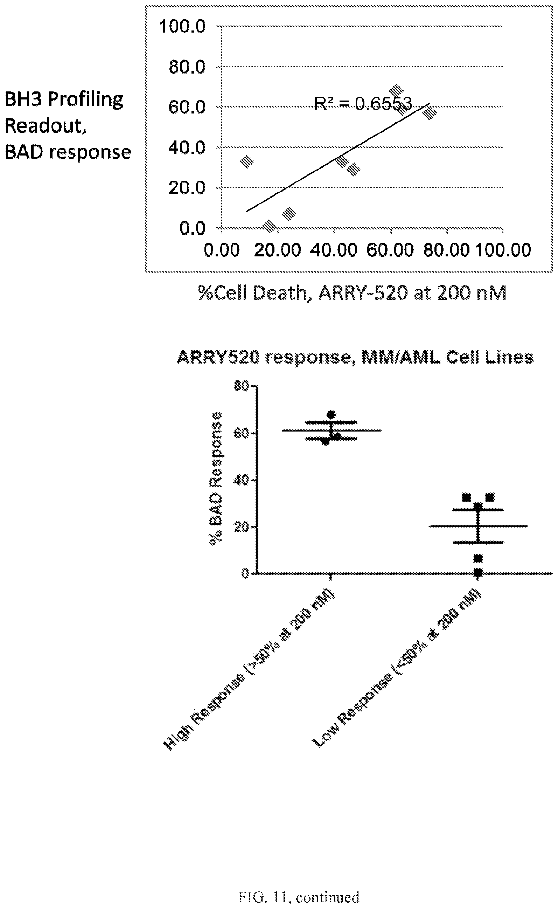

*Kinesin Spindle Protein (KSP) inhibitor development:* A KSP inhibitor was tested in antiproliferative assay against 8 multiple myeloma/leukemia-derived human cell lines. BH3 profiling was simultaneously performed on these cell lines. The data yields a strong indication of correlation between BH3 profiling readout (% priming with respect to PUMA and BAD, individually) with antiproliferative properties of the compound (**FIG. 11**).

Correlation of KSP inhibitor activity may be modulated by levels of MCL1 protein. If this were the sole mechanism, one may expect that Noxa, as a regulator of MCL1, would be an adequate predictor of efficacy. In fact, an unexpected result was obtained in that independently PUMA (modulation of MCL1, BCL2 and BCLxl) and BAD (effector of BCL2 and BCLxl) were able to discriminate therapeutic efficacy in these cell lines.

Kinesin Spindle Protein inhibitors regulate anti-cancer activity by interaction with and re-modeling of microtubule architecture. Such a mechanism of action makes it surprising that that the action of these agents could be predicted by mitochondrial response and subsequent apoptosis signaling.

## Example 2: Studies using oncology patient-based cohorts

*AML (cytarabine-based treatment [standard-of-care]), methodology: AML Patient Cohort:* Newly diagnosed AML patient samples were obtained either by peripheral blood draw or bone marrow aspirate (BM) collection prior to induction chemotherapy administration between September 1999 and March 2007.[39] Specimens were acquired during routine diagnostic assessments in accordance with the regulations and protocols (Lab 01-473) approved by an investigational review board. Informed consent was obtained in accordance with Declaration of Helsinki. Following Ficoll purification, CD3/CD19 cell depletion removed contaminating T and B cells. Individual aliquots of cells were centrifuged and resuspended in 90% FBS/10% DMSO and cryopreserved in liquid $N_2$. Pathologic classification, cytogenetic analyses, and mutational status were obtained; clinical indicators are shown in supporting tables and figures.

*Patient Treatment:* Patients were classified for response per standard criteria. Of the 62 patients, 48 were treated with cytarabine + anthracycline, 7 patients with cytarabine + non-anthracycline and 8 patients with cytarabine + fludarabine (one patient was treated with cytarabine + non-anthacycline and then cytarabine + fludarabine on a subsequent cycle [no response on either cycle]). CR= Normal bone marrow morphology, absolute neutrophil count greater than 1,000, platelet count >100K and rising hemoglobin. Primary refractory = residual leukemia after 2 cycles of induction chemotherapy (could be same or different regimens). Relapse is >5% blasts in the marrow or blast in the peripheral blood in a patient formerly in CR.

*Cytogenetic Risk Status Determination:* Cytogenetic risk determination was performed by a CLIA certified cytogenetics lab. In brief, patients were classified according to standard grouping: Favorable = inv16, t(8;21), T(15;17) intermediate = diploid, -y, insufficient metaphases, Unfavorable = all others , -5, -7, +8, t(6;9), 11q, PH1+, misc.

*BH3 Profiling:* Ficoll-purified, viably frozen, pre-treatment AML specimens were thawed, resuspended in FACS buffer (1%FBS, 2mM EDTA, PBS) with FCR blocking reagent (Miltenyi Biotec, Auburn CA) for 10 minutes on ice and then stained with antibodies CD45-V450 (BD Biosciences, San Jose CA), CD3-Biotin (BD Bioscience, San Jose CA), and CD20-Biotin (eBiosciences, San Diego CA) for 20 minutes on ice. Samples were re-suspended in FACs buffer with secondary antibody Streptavidin-APC (BD Biosciences, San Jose CA) for 20 minutes on ice. Following staining, AML specimens were permeabilized with digitonin (Sigma-Aldrich, St Louis MO) and incubated for 180 minutes with peptides (BIM 100μM, BIM 0.1μM, PUMA 100μM, PUMA 10μM, NOXA 100μM, BAD 100μM, BMF 100μM, HRK 100μM, or PUMA2A 100μM) or with dimethyl sulfoxide (DMSO [(1%]) or Carbonyl cyanide m-chlorophenyl hydrazone (CCCP [10μM]) at $2 \times 10^5$ cells per tube in Newmeyer Buffer (80mM KCl, 10mM HEPES, 40μM EDTA, 40μM EGTA, 5mM Succinate, 300mM Trehalose, 0.1% BSA, pH 7.4) at room temperature. Samples were run in duplicate except in cases where insufficient viable cells were available. Potentiometric JC-1 mitochondrial dye (Enzo Life Sciences, Farmingdale NY) was added 45 minutes prior to analysis.

Samples were analyzed on a FACS CantoII (BD Biosciences, San Jose CA) using the BD FACS Diva software. The blast population was identified as CD45 dim, SSC low, CD3 and CD20 negative. Intensely stained CD45 cells representing mature lymphocytes were excluded from analyses as described previously. The quantifiable propensity of a pro-apoptotic peptide to induce mitochondrial depolarization relative to an uncoupling reagent control is referred to as percent priming. For the blast population this was calculated using the median signal intensity of the PE channel normalized for DMSO as background (negative control) and CCCP served as 100% priming (positive control).

*Statistical Analysis:* Predictive values of BH3 profiling biomarkers were studied by testing the association between the biomarker status (% priming) and whether the patient was characterized as a responder or non-responder. Univariate comparisons were made using the Mann-Whitney test and all reported p-values are two-sided. A statistical analysis plan with a threshold for significance of p<0.01 to limit the risk of false-positive results (p values >0.01 and <0.05 were considered as borderline significant) was pre-determined. The predictive ability of markers was assessed using the area under the curve (AUC) statistic. Survival endpoints were analyzed using Cox proportional hazards regression. Multivariate analyses were performed using logistic regression, and used adjustment variables that were significant from patient clinicopathologic information using the above criteria. OS and EFS were tested for significant correlation with percent priming by logrank test for trend. Analyses were performed using SAS software, version 9.2 (SAS Institute Inc., Cary, NC), R version 2.14.2 (R Core Team; Vienna, Austria), and/or Graphpad Prism version 5.04 (La Jolla, CA).

*R Patient Cohort Characteristics:* AML patients were stratified by cytarabine-based regimen response status relative to clinical pathologic variables (**Table 3**). Mann-Whitney analyses were performed to test for nonrandom association of clinical variables and chemotherapeutic response. Of variables tested, only patient age profile and cytogenetic risk stratification displayed nonrandom association relative to response (P=0.008 and P=0.006, respectively). These two non-random variables were subsequently utilized in multivariate analyses described below for BH3 profiling biomarkers.

*BH3 Profiling of Patient Specimens:* Of the 62 viably preserved AML patient specimens that were BH3 profiled as part of this study, 61 provided analyzable data. The one sample that was eliminated from consideration prior to statistical analysis yielded a profile by which insufficient viable cells were identified by Trypan Blue exclusion to continue with analysis. That 61 of 62 patient specimens were able to be assayed by BH3 profiling indicates an overall technical success of 98.4%. Further, the technical failure we associated with this specimen apparently is consistent with a compromised freeze as poor cell viability was noted immediate upon thawing.

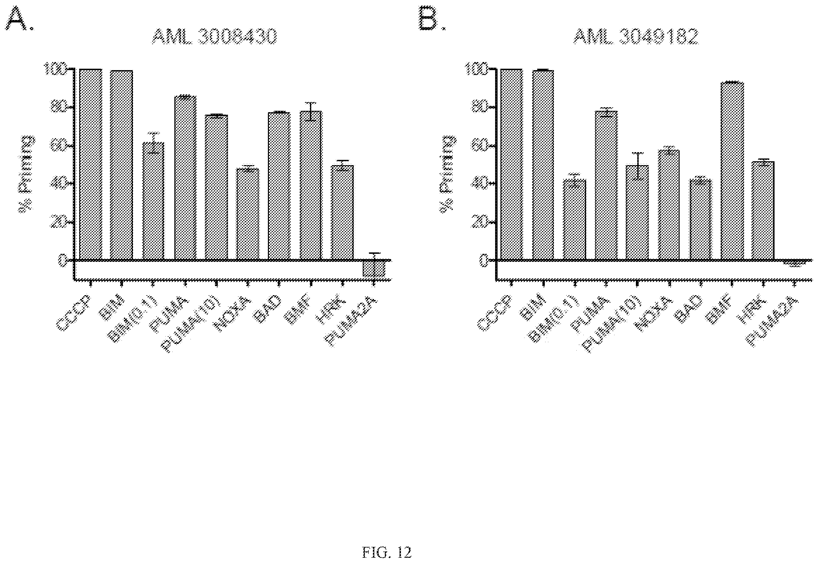

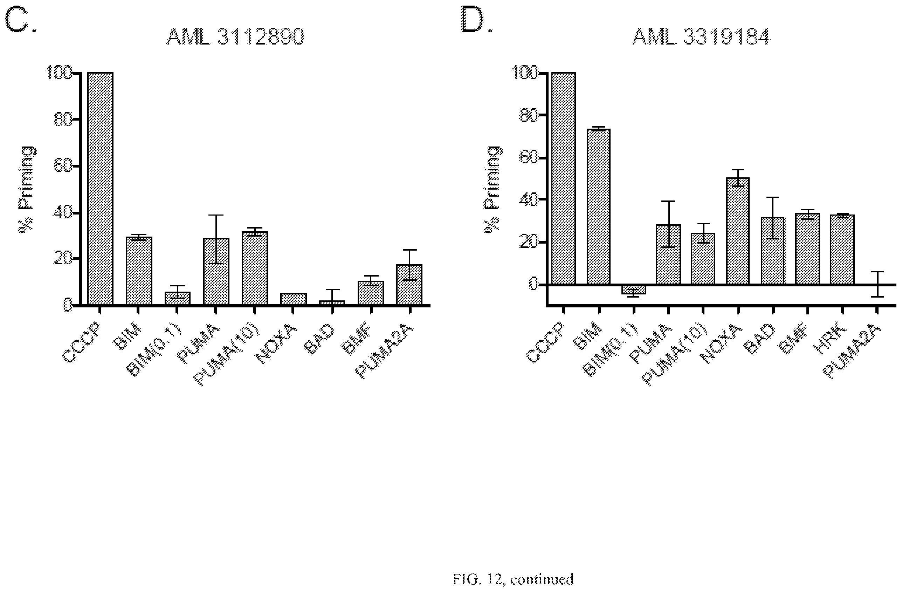

All peptides have been empirically optimized to give a dynamic range of percent priming values prior to initiation of these studies with previous AML specimens. Most notable is that 100 $\mu$M BIM was found to be saturating (or near saturating) for a majority of AML patient specimens. Thus, in addition to 100 uM BIM, BIM was also assayed at 0.1 $\mu$M, a concentration that has been determined to provide a dynamic range of percent priming values. Representative data is shown in **FIG. 12** of two NR and two CR patients. Note that the overall Coefficient of Variation (CV) for repeat samples from individual patients is generally 3-5%, indicative of a technically robust assay with limited run-to-run variability.

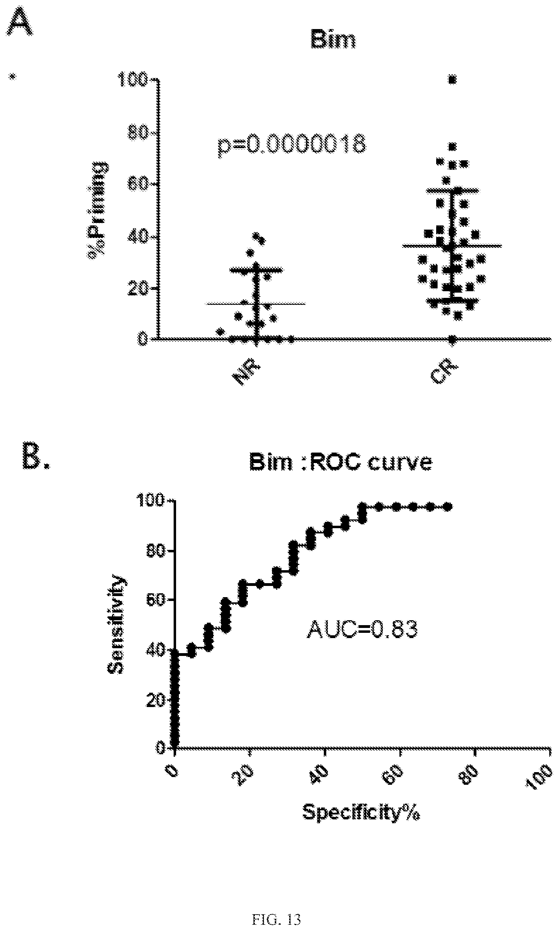

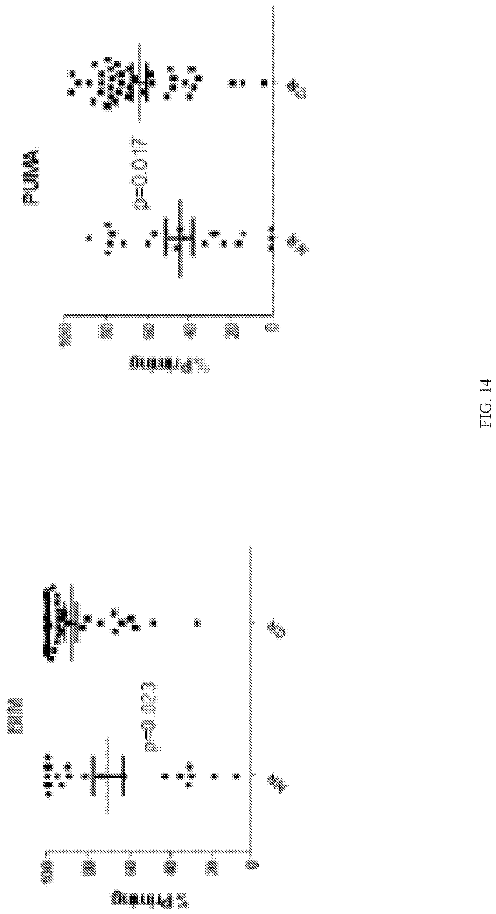

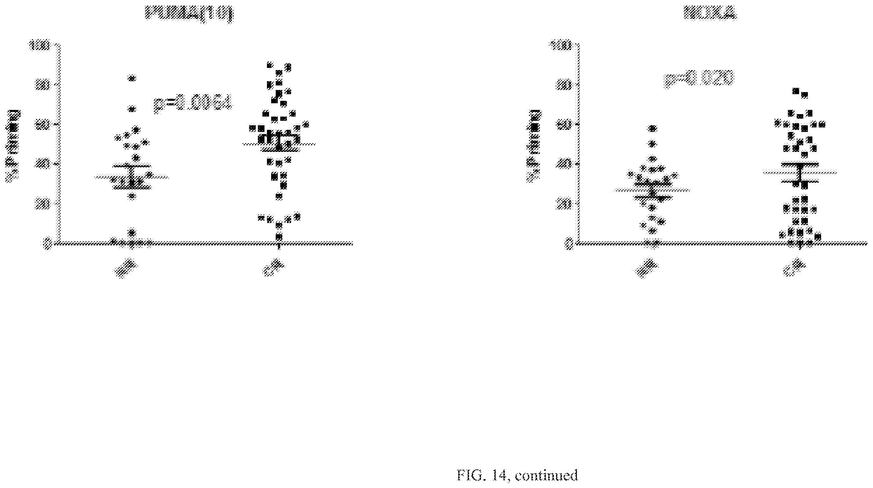

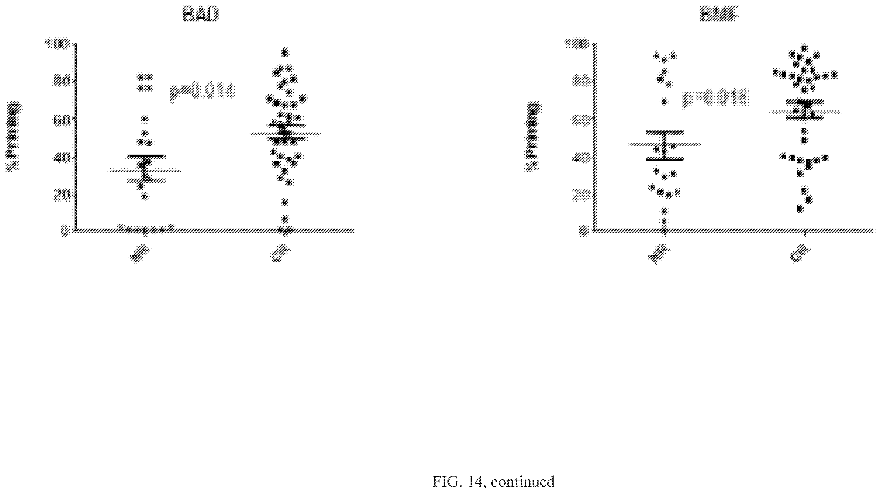

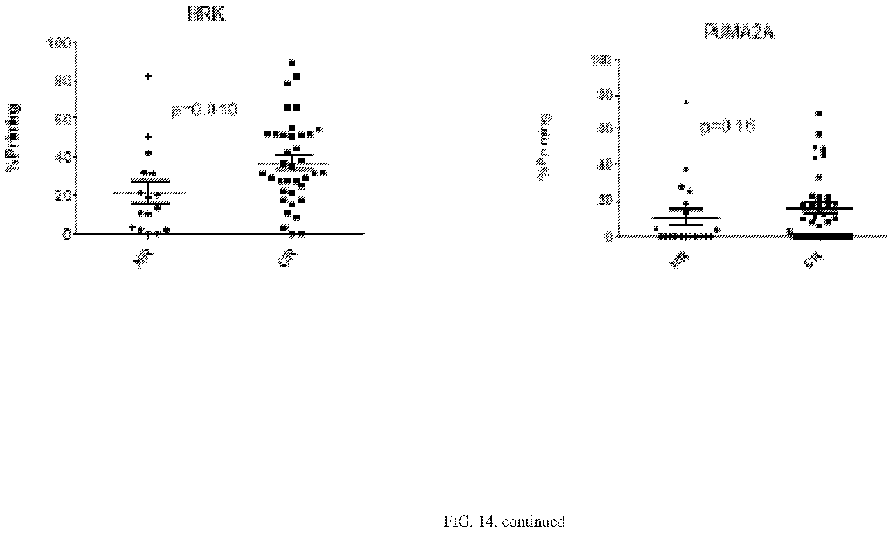

Among the biomarker peptides assayed, higher BIM (0.1) percent priming scores correlated with response at a high degree of statistical significance (p=0.0000018) (**FIG. 13**). Analyses of other BH3 Profiling biomarkers assayed are indicated in **Table 4.** In addition to statistical significance yielded by BIM(0.1), PUMA(10) displayed significant association with response (p=0.0064) (**FIG. 14**).

When the BIM(0.1) priming scores of individual patients were segregated into responder and non-responder groups (**FIG. 2**), a clear trend emerged. AML patients likely to exhibit response to cytarabine-based therapy tend to have higher BH3 profiling priming (percent priming = 36.8 $\pm$ 21.2[SD]) than those patients not likely to respond (percent priming = 13.2 $\pm$ 13.4[SD]). In establishing sensitivity and specificity of this biomarker in correctly identifying the likely responders while at the same time correctly separating the non-responders, receiver operator characteristic (ROC) plot depiction indicates an AUC of 0.83 [95%CI:0.73,0.94] (**FIG. 14**), an outstanding indication of the ability of the biomarker to correctly discriminate individual specimens. Interestingly, within these numbers, a single biomarker may achieve identification of 89.7% of responders while at the same time 59.1% of those patients unlikely to response. If the desired sensitivity cut-off is a little higher at 92.3%, then the specificity still achieves 54.6% of unlikely responders.

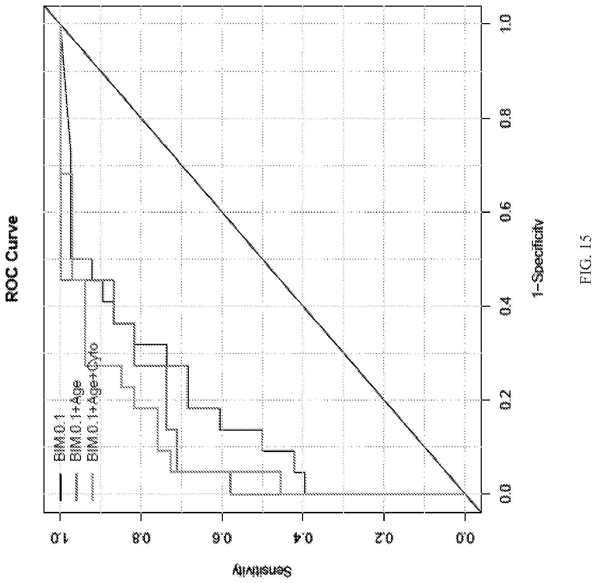

Age and cytogenetics were shown to be prognostic factors in AML in this dataset as well (**Table 3**). To determine if the addition of BIM(0.1) % priming biomarker added prognostic information beyond that of age profile and cytogenetics, age profile and cytogenetics were serially added to BIM (0.1)% priming multivariate analyses. The addition of patient age profile to BIM(0.1) yields an increase in the AUC to 0.89 from the previous BIM(0.1) AUC=0.83 alone (**FIG. 15**). Further, when BIM(0.1) is adjusted for patient age profile and cytogenetic risk, then the AUC further increases to 0.91. Within this latter adjustment, >90% sensitivity is achieved with identification concurrent with segregation of >70% of the likely non-responders (**FIG. 15**).

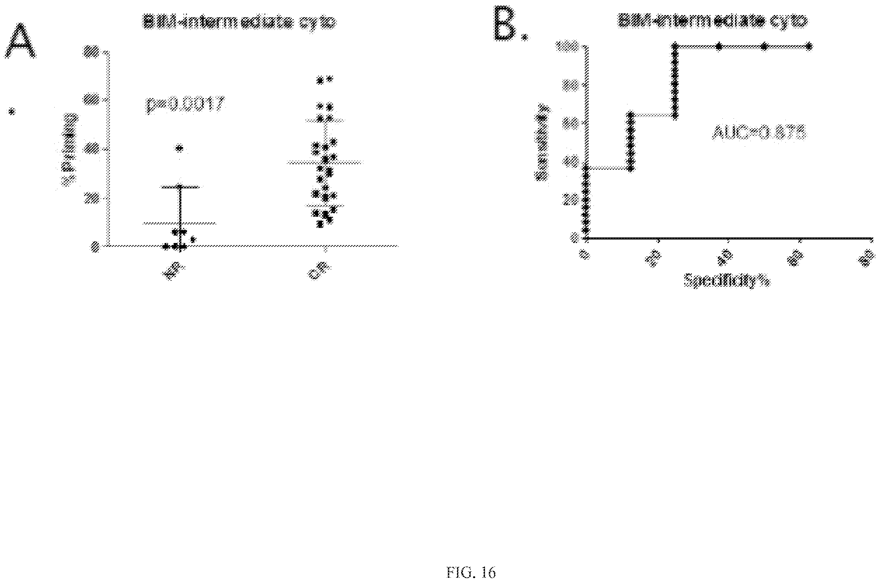

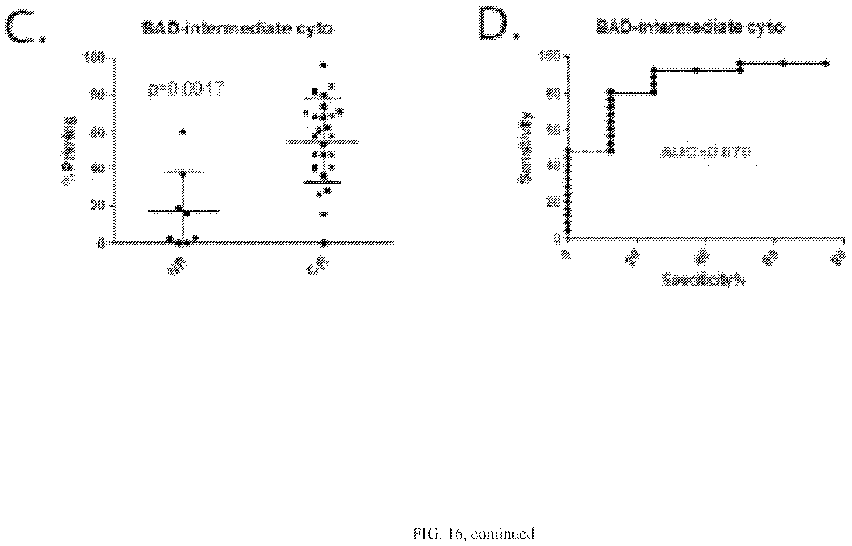

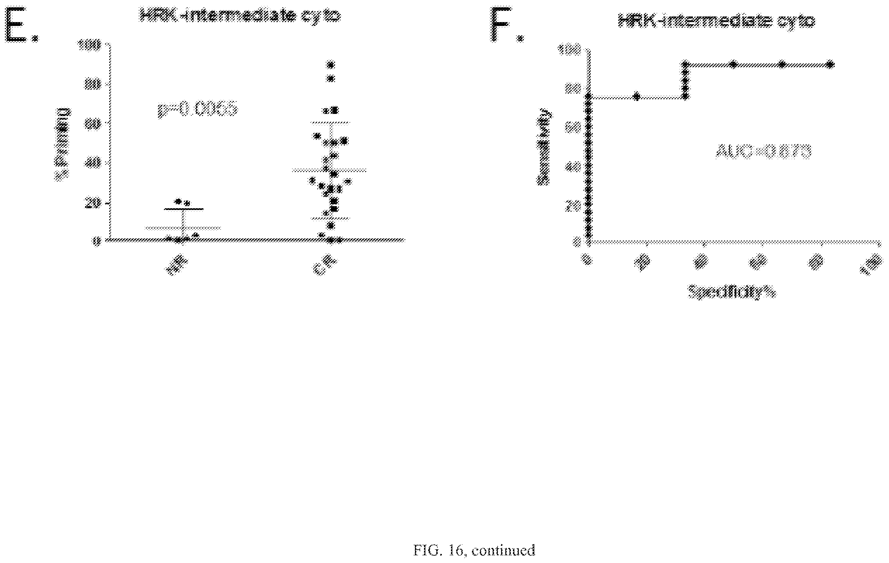

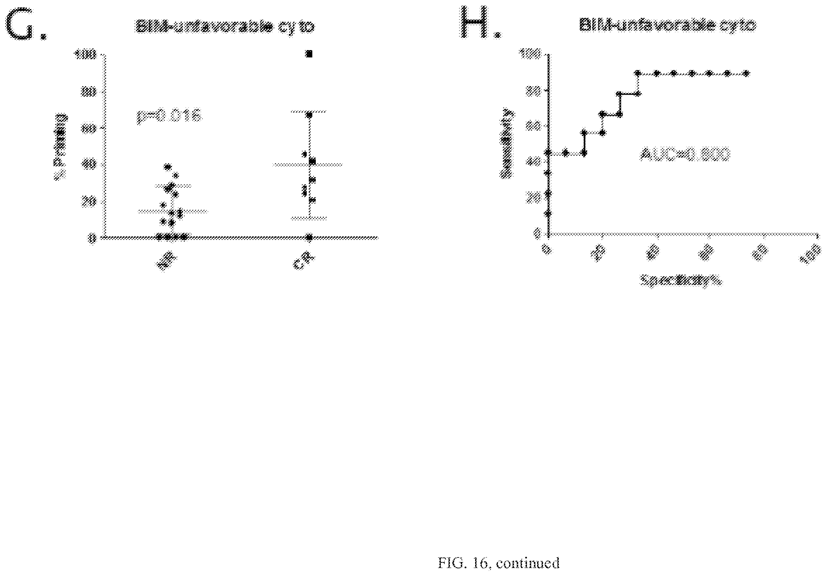

Patients were stratified by cytogenetic risk status and then these subgroups were analyzed by Mann-Whitney for significance in identifying responders and non-responders. In the intermediate risk (n=33) sub-group, BIM(0.1) was very significantly associated (p=0.0017) with further discriminating response and in the unfavorable group (n=23) BIM(0.1) was still significant (p=0.023) (**FIG. 16**). While the p-values here are somewhat diminished relative to BIM(0.1) analysis of the combined cohort, this is generally a phenomenon of reduced statistical power owing to the sub-grouped number of patients. Interestingly, both BAD and HRK analysis yielded interesting significant p-values in response discrimination (p=0.0017 and p=0.0055, respectively); however, this was only observed for the intermediate risk group (**FIG. 16**). Sensitivity and specificity assessment by ROC analyses of these biomarkers in response discrimination gives AUCs of 0.875 for BIM(0.1), 0.875 for BAD, and 0.823 for HRK in the intermediate group and 0.790 for BIM(0.1) for the unfavorable group (**FIG. 16**). It should be noted that these AUCs may benefit from somewhat imbalanced subgroupings for responders versus non-responders in the independent sub-groups (8 NR, 25 CR for intermediate and 15 NR and 9 CR for unfavorable). As there were only data for 5 favorable patients, statistical analysis was not possible on this group.

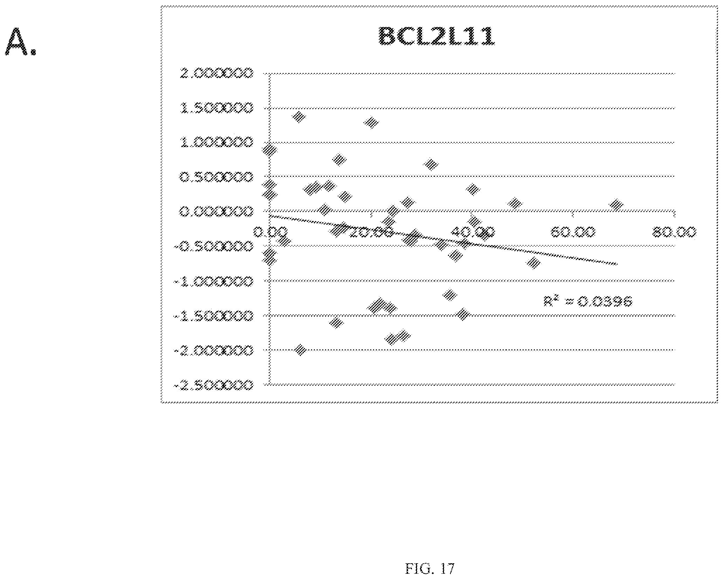

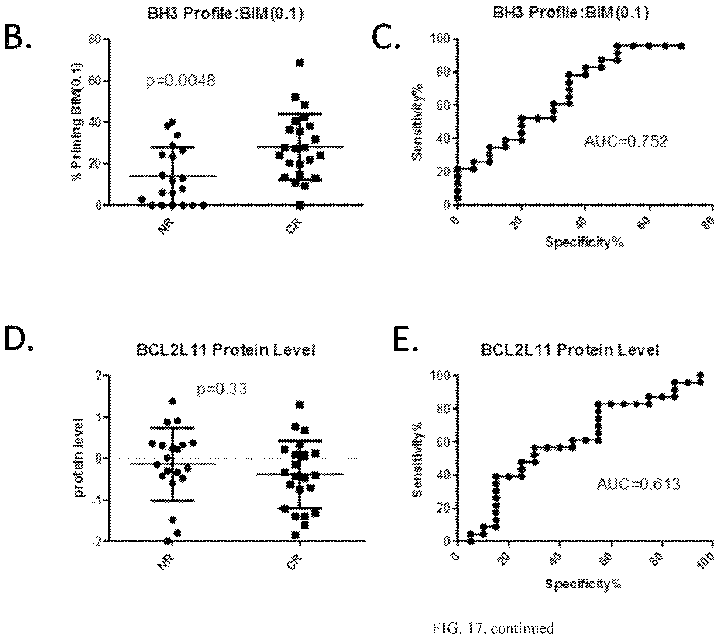

*Comparison of BIM (0.1) BH3 Profiling Percent Priming and BIM (BCL2L11) Protein Levels:* In order to assess whether the predictive power of BIM BH3 profiling is merely re-capitulating BIM protein levels, an assessment of whether a correlation or lack thereof exists in the AML patient specimens within this study was undertaken. It was found that no correlation exists between BIM protein level and percent priming (**FIG. 17**) yielding an $R^2=0.0396$.

BH3 profiling of BIM(0.1) maintains a significant p-value (p=0.0048) (**FIG. 17**) in this subset for which both BH3 profiling and RPPA data exist of the total patients cohort. Note here that the power of the analysis is reduced relative to our earlier analyses as sample size is diminished from an n=62 to an n=43 and many of the samples that did not have RPPA data were among the highest scoring BH3 Profiling specimens. The p-value for response discrimination for this same subset of specimens for BCL2L11 protein level is p=0.33 (**FIG. 17**). These data provide strong evidence that BH3 profiling is not correlated with overall protein levels and that BH3 profiling may offer a new paradigm by which to predict cytarabine response in AML patients.

## Example 3: Secondary Clinical Endpoints: Overall Survival and Event-free Survival

BH3 profiling biomarkers were also analyzed for correlation to the secondary clinical endpoints overall survival (OS) and event-free survival (EFS). Continuous variable models using Cox Proportional analyses indicated that BIM(0.1) was not significant for EFS (p=0.14) or OS (p=0.057). Cox Proportional Hazard Analysis between NOXA percent priming and EFS also were non-significant p=0.089. All other peptides tested yielded no significant correlation or trends between either OS or EFS and % priming (all p>0.10). Further, multivariate analysis with adjustment variables patient age profile and cytogenetic risk status failed to yield significant correlations between BH3 profiling biomarkers and OS and EFS clinical endpoints.

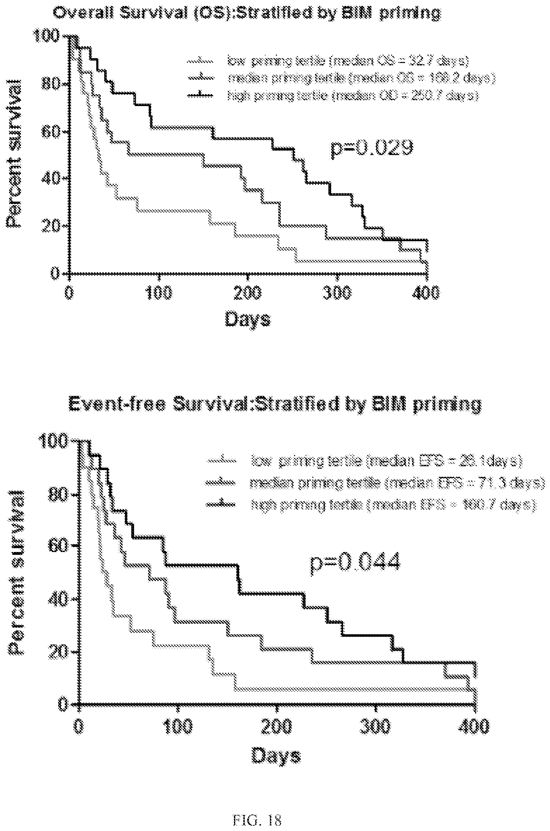

Interestingly, in partition model analyses, when the patient cohort was divided into tertiles by BIM percent priming (High Priming, Intermediate Priming, and Low Priming), corresponding OS yielded a median of 250.7, 168.2 and 32.7 weeks, respectively (p=0.029, logrank test for trend) (**FIG. 18**). When the same analysis of these tertiles was conducted for EFS, median EFS was 26.1, 71.3, and 160.7 weeks for low priming, intermediate priming, and high priming tertiles, respectively (p=0.044, logrank test for trend) (**FIG. 18**).

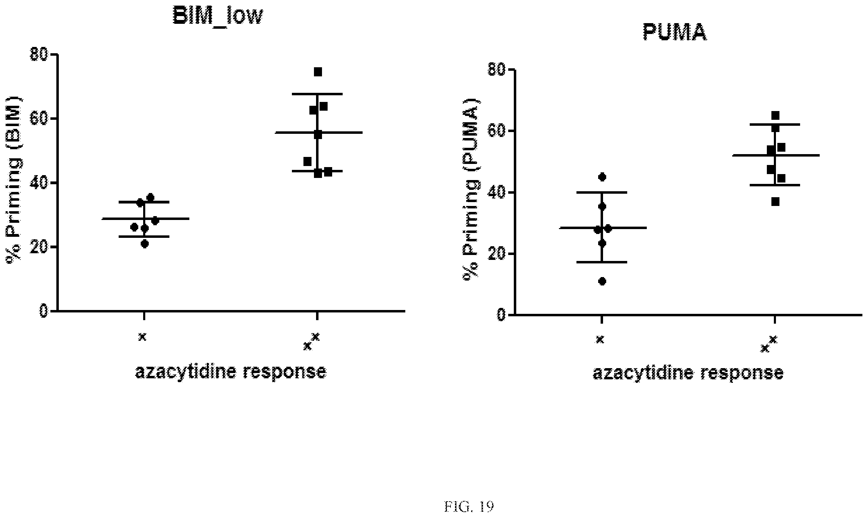

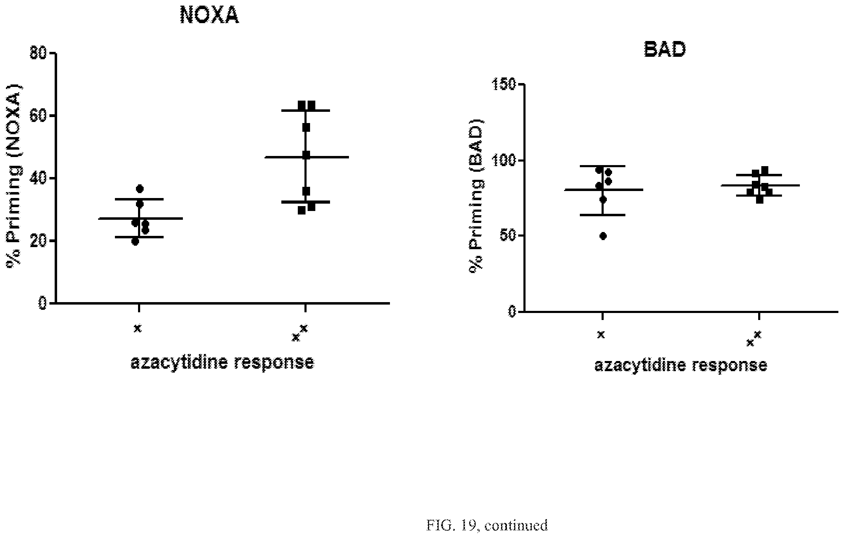

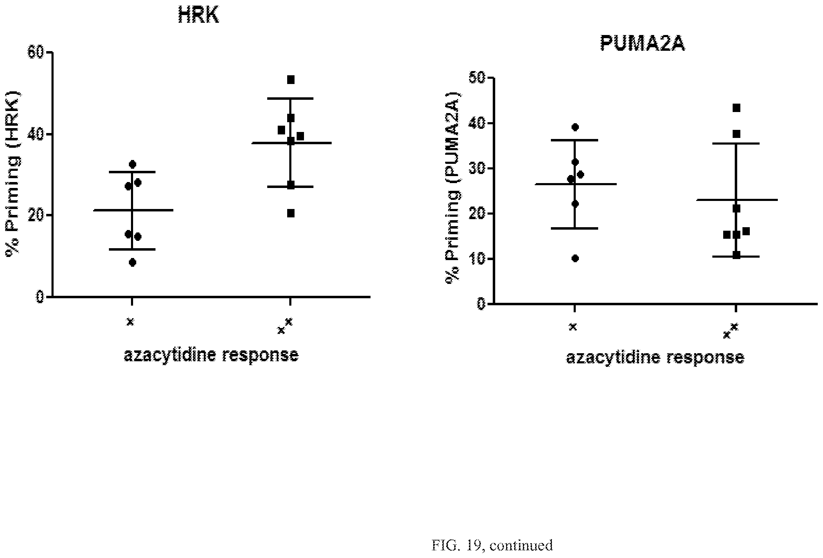

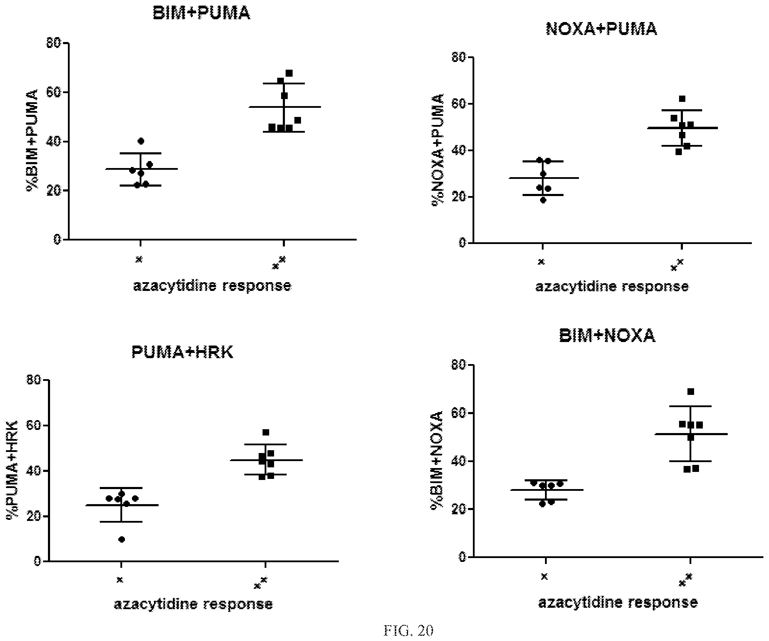

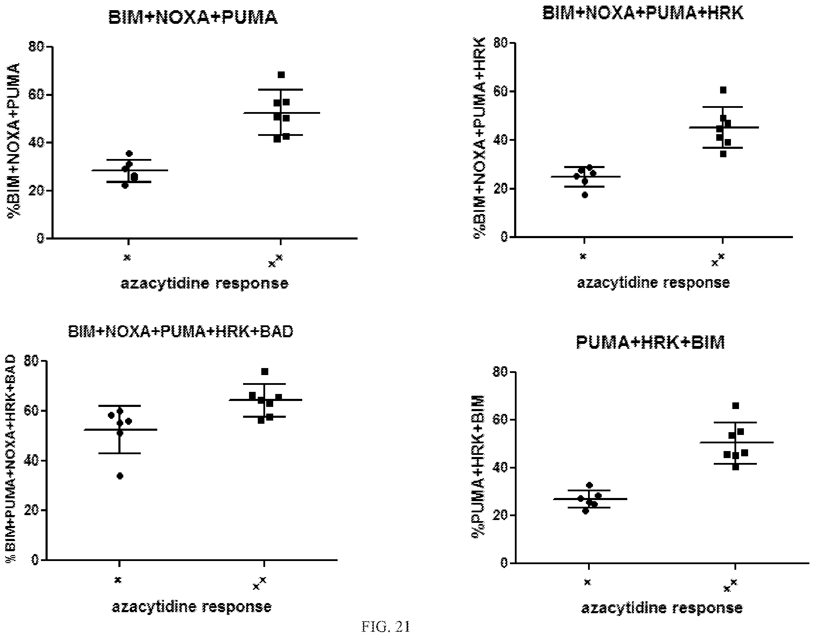

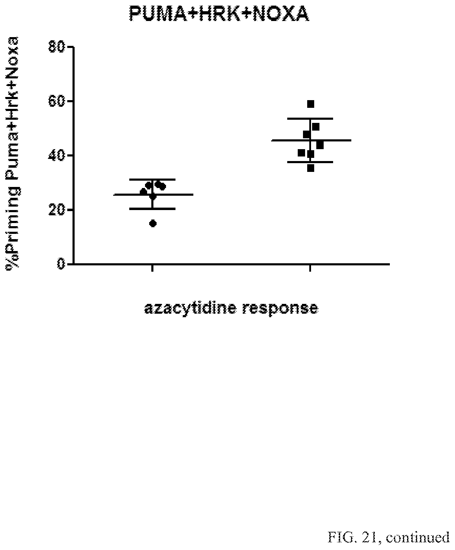

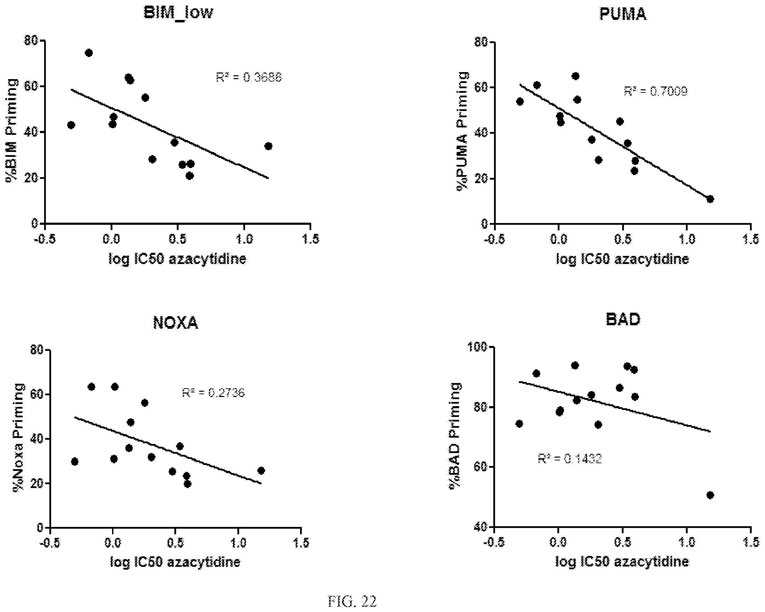

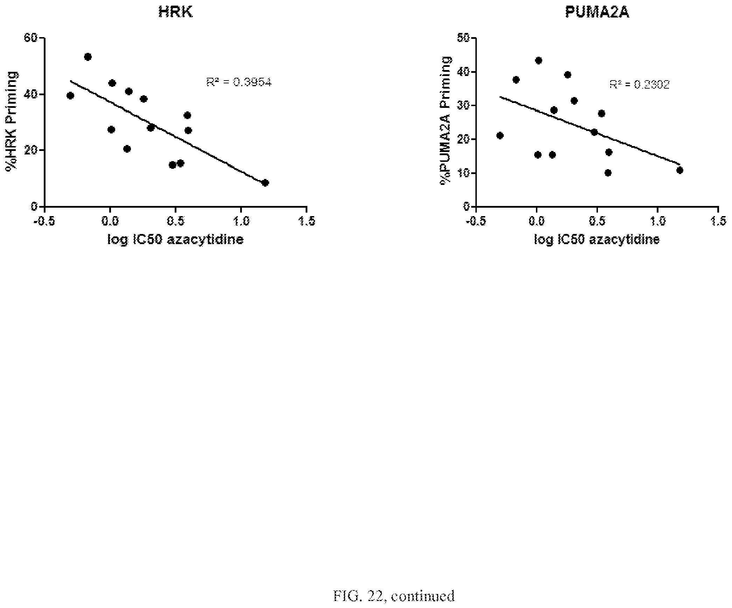

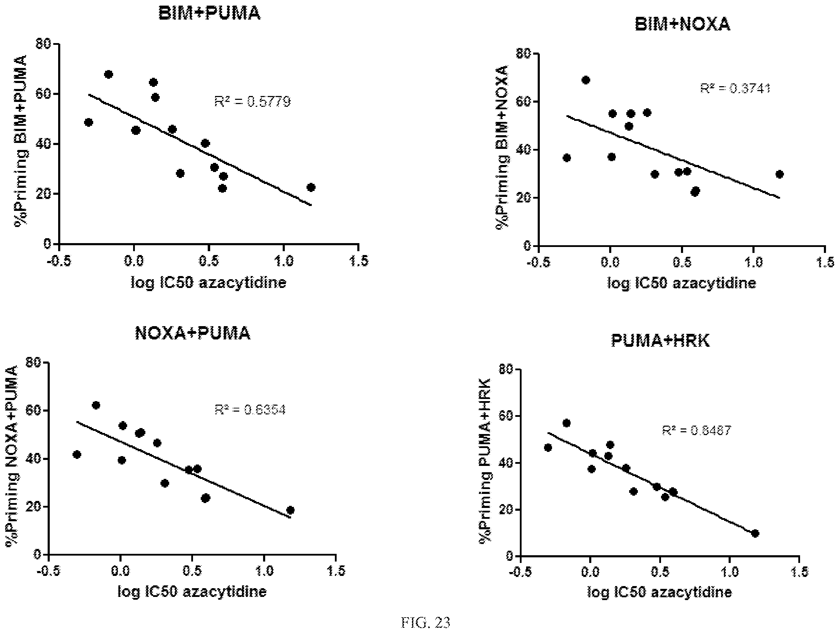

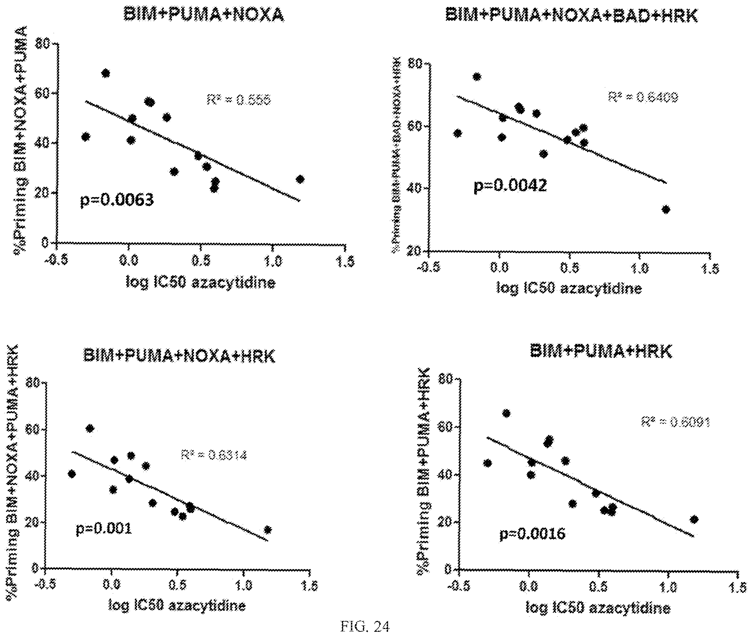

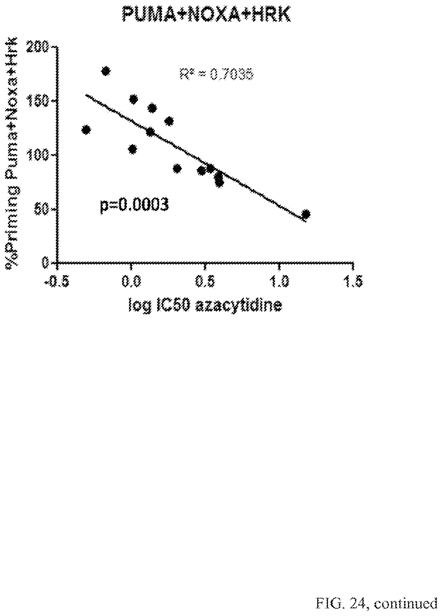

*AML and Azacytidine:* Thirteen human AML derived cell lines were BH3 profiled and correlative analyses performed for in vitro azacytidine response. Partition models utilizing BH3 metrics discriminated azacytidine response with statistical significance (p<0.01) between more sensitive (IC50<2 uM) and less sensitive (IC50>2uM) AML-derived cell lines using individual peptide-derived models (**FIG. 19**) as well as two peptide models (**FIG.** 20) and models comprising 3 or more peptides (**FIG. 21**). Using continuous variable analysis, $R^2 > 0.7$ for individual peptide-derived algorithms (**FIG. 22**) and combined BH3 peptide models relative to $_{[log]}$IC50s comprising two peptides (**FIG. 23**) and three or more peptides (**FIG. 24**) when Puma (pan-priming indicator) is employed. Statistically significant p-values mostly track with models including Puma (p<0.01). Results are summarized in **Table 5.**

As azacytidine belongs to class of anti-cancer agents known as epigenetic modifying agents, a direct modulation of apoptosis and effect on mitochondrial biology is unlikely. Therefore, it is surprising that azacytidine therapeutic efficacy may be predicted by metrics designed to interrogate the intrinsic apoptosis pathway and mitochondrial biology. *See, e.g.,* Vo et al. (Cell. 2012; 151(2):344-355) which reported that azacytidine efficacy was not predicted by BH3-derived metrics.

The data were compelling to proceed with examination of primary AML patient specimens for the purpose of BH3 profiling for modeling azacytidine outcomes.

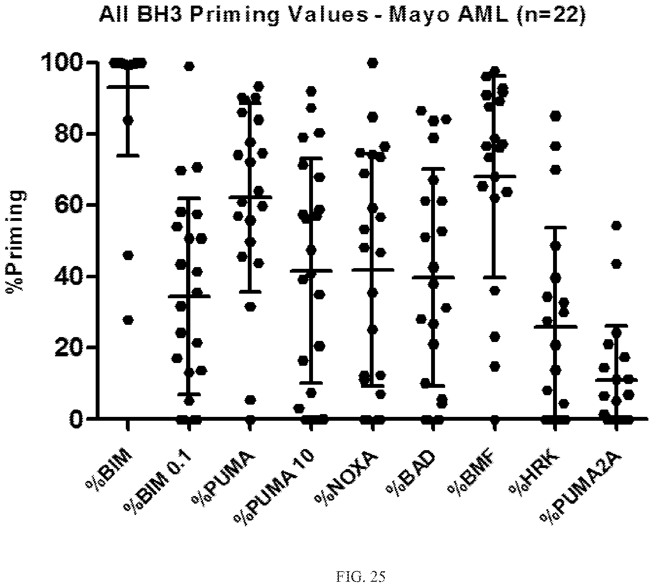

Supporting data for algorithms derived from BH3 metrics from azacytdine-treated AML patients has been generated. In a study comprising an N= 28 combined cohort (13 (9 stable/CR; 4 refract/NR) specimens and 15 (all NR/refract)). One specimen was not evaluable. In all, 27 specimens were analyzed relative to response (19 NR, 8 R). The range of reported scores for this cohort is indicated in **FIG. 25** and illustrates that the range of scores provides for a therapeutic window against which response and other clinical endpoints such as overall survival (OS) and event-free survival (EFS) may be measured.

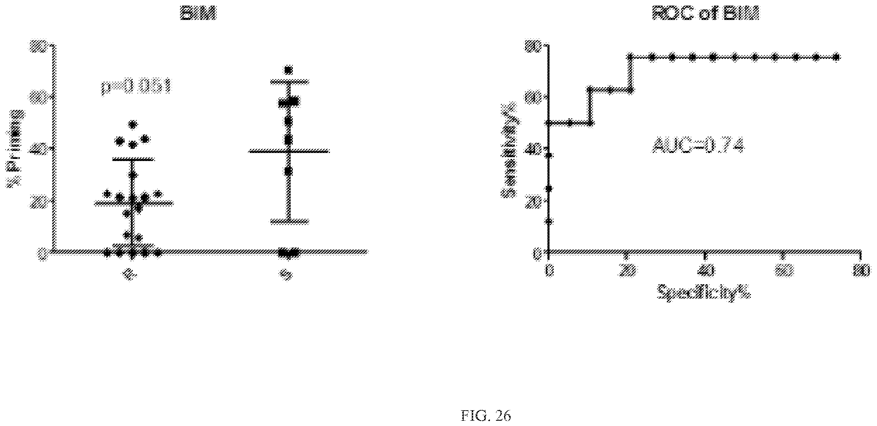

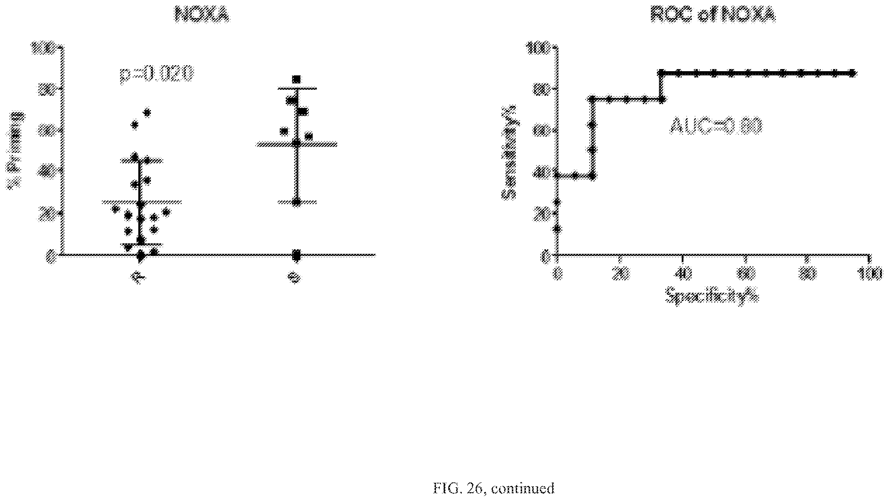

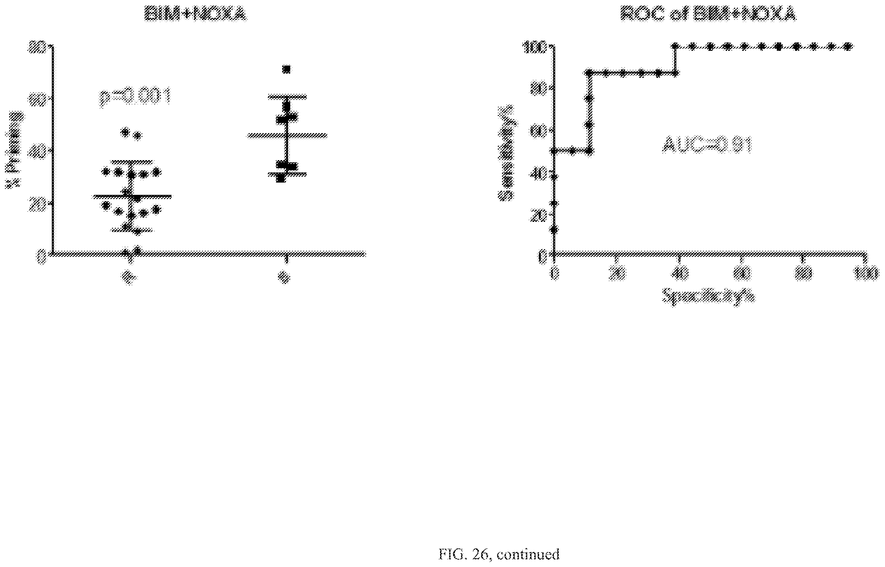

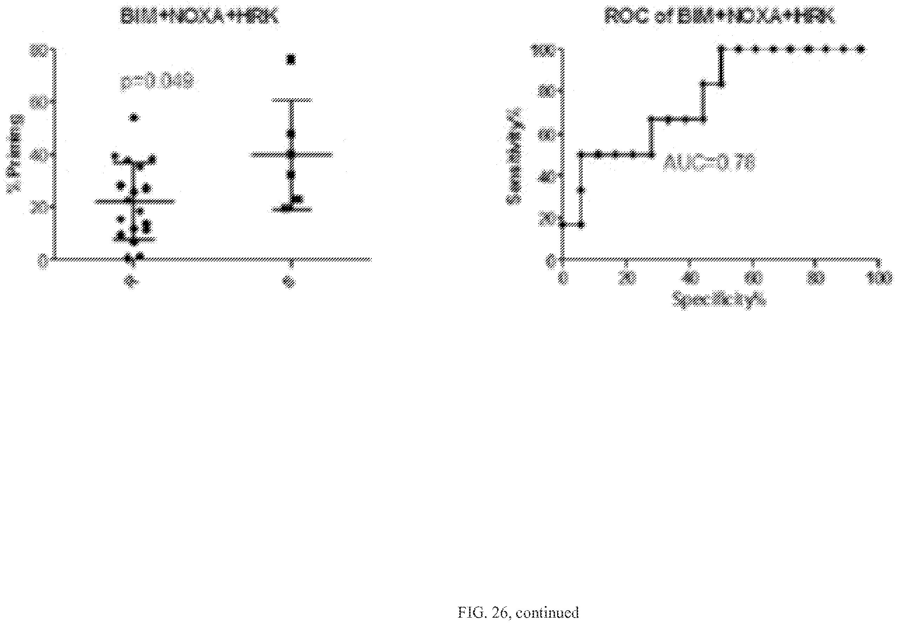

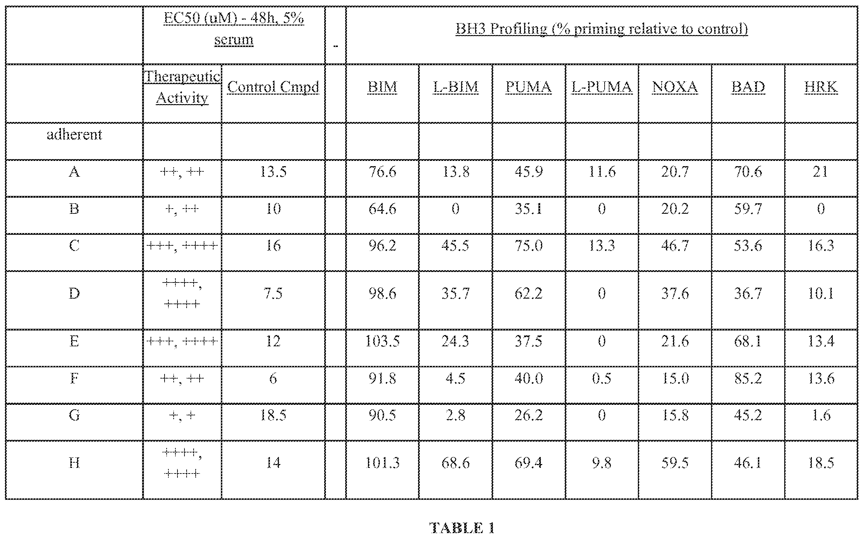

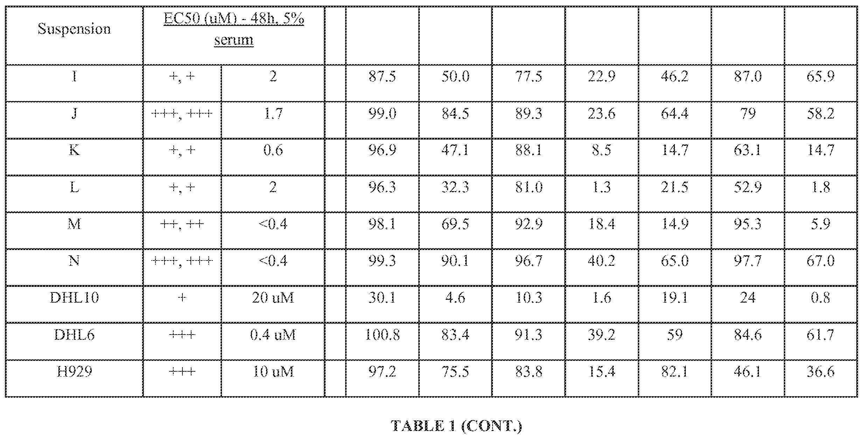

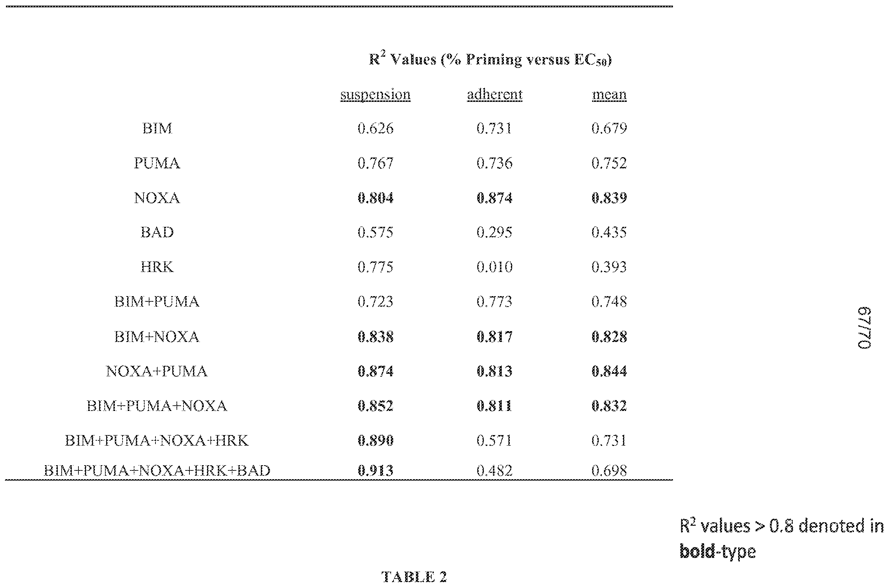

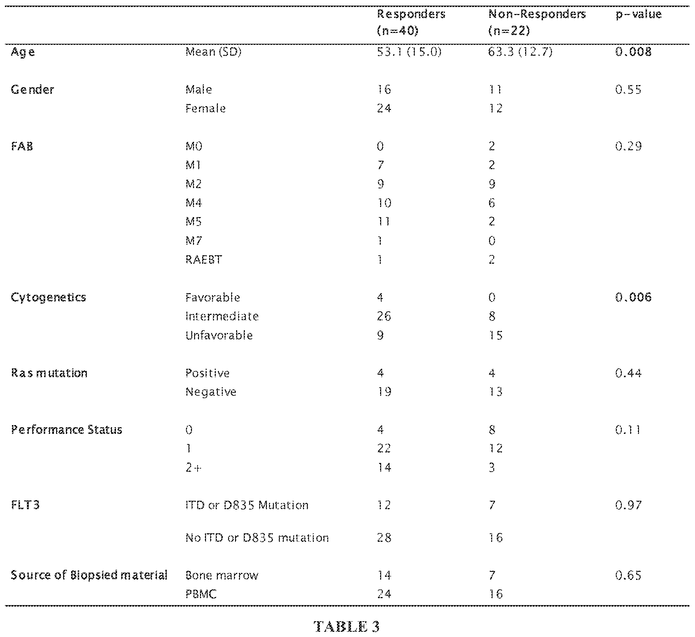

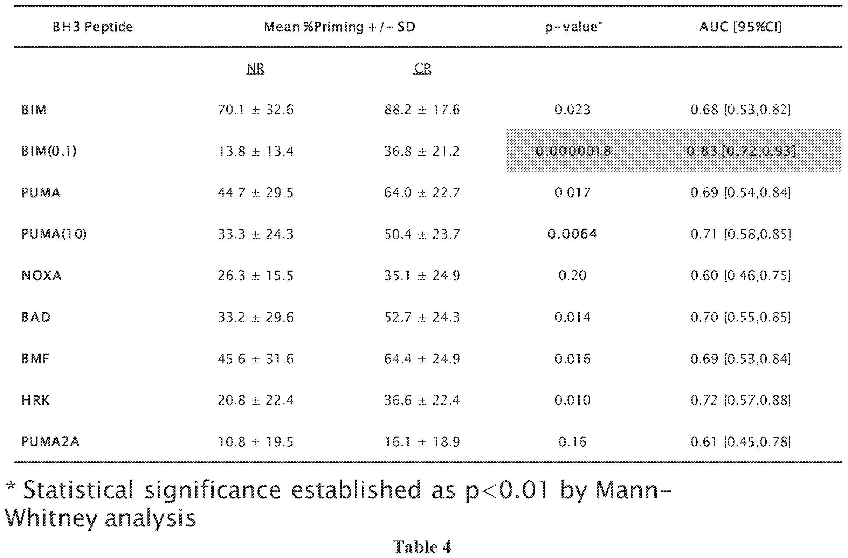

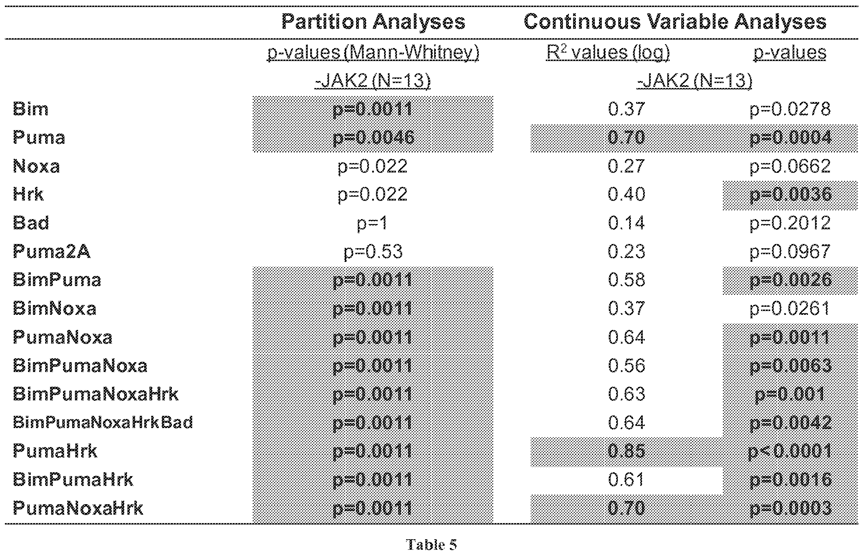

For individual markers, only BIM and NOXA yielded borderline significant association with response (p=0.05 and p=0.02, respectively). All other biomarker p-values>0.1. However, when BIM and NOXA were combined the presently described approach, correlation with response was highly significant (p=0.001). Further, the ROC for sensitivity/specificity was 0.91. This is univariate analyses only and may be stronger statistically when clinical adjustment variables are weighed (*e.g.*, age, cytogenetic status, etc) (**FIG. 26**).

**Claims**

1. A method for determining a cancer treatment for a patient, comprising:

   determining a BH3 profile for the patient's cancer cell specimen, wherein the cancer is selected from acute myelogenous leukemia or multiple myeloma, and wherein determining the BH3 profile comprises permeabilizing the patient's cancer cells, determining a change in mitochondrial membrane potential upon contacting the permeabilized cells with one or more BH3 domain peptides; and correlating a loss of mitochondrial membrane potential with chemosensitivity of the cells to apoptosis-inducing chemotherapeutic agents;
   determining one or more clinical factors of the patient selected from the group consisting of age, cytogenetic status, performance, histological subclass, gender, and disease stage, and
   classifying the patient for likelihood of clinical response to one or more cancer treatments,
   wherein the likelihood of the clinical response is determined by assessing a percent priming, wherein the percent priming measures the response of the cancer cell specimen to said contacting with one or more BH3 domain peptides and the percent priming is defined by the following equation:

$$\%Priming = \left[100 \times \left(\frac{DMSO\ AUC - Peptide_1\ AUC}{DMSO\ AUC - CCCP_{avg}AUC}\right)\right]Peptide_1 + \left[100 \times \left(\frac{DMSO\ AUC - Peptide_2\ AUC}{DMSO\ AUC - CCCP_{avg}AUC}\right)\right]Peptide_2 + \cdots / (n\ peptides)$$

   wherein:

   AUC is either

   (i) the area under a curve of a homogenous time-resolved fluorescence (HTRF) measurement, or
   (ii) the mean signal intensity of a fluorescence activated cell sorting (FACS) measurement at a single time point that occurs between about 5 min and about 300 min after the start of said contacting,

   of said change in mitochondrial membrane potential;
   DMSO (dimethyl sulfoxide) is a baseline negative control; and
   CCCP (Carbonyl cyanide *m*-chlorophenyl hydrazone) is a chemical inhibitor of oxidative phosphorylation and comprises an effector of protein synthesis by serving as uncoupling agent of the proton gradient established during the normal activity of electron carriers in the electron transport chain in the mitochondria, and is a baseline positive control;
   Peptide is said one or more BH3 domain peptides;

   wherein the one or more clinical factors are selected to increase specificity and/or sensitivity of the BH3 profile for association with clinical response.

2. The method of claim 1, wherein the cancer treatment is one or more of a BH3 mimetic, epigenetic modifying agent, topoisomerase inhibitor, modulator of cell cycle regulation, and kinesin-spindle protein stabilizing agent.

3. The method of claim 2, wherein the modulator of cell cycle regulation is a cyclin-dependent kinase inhibitor.

4. The method of claim 1, wherein determining the BH3 profile comprises use of the BH3 domain peptide, wherein the peptide is one or more of BIM, BIM2A, BAD, BID, HRK, PUMA, NOXA, BMF, BIK, and PUMA2A.

5. The method of claim 1, further comprising measuring the BH3 profile and one or more of a cell surface marker CD33, a cell surface marker CD34, a FLT3 mutation status, a p53 mutation status, a phosphorylation state of MEK-1 kinase, and phosphorylation of serine at position 70 of Bcl-2; and correlating to efficacy in treating AML patients with cytarabine or cytarabine-based chemotherapy and/or azacytidine.

**Patentansprüche**

1. Ein Verfahren zur Bestimmung einer Krebsbehandlung für einen Patienten, umfassend:

Bestimmung eines BH3 Profils für das Krebszellspezimen des Patienten, wobei der Krebs ausgewählt ist aus Akuter Myeloischer Leukämie oder Multiplem Myelom, und wobei das Bestimmen des BH3 Profils die Permeabilisierung der Krebszellen des Patienten, Bestimmung einer Veränderung des mitochondrialen Membranpotenzials nach Kontaktierung der permeabilisierten Zellen mit einem oder mehreren BH3 Domänen-Peptiden und das Korrelieren eines Verlusts an mitochondrialem Membranpotenzial mit der Chemosensitivität der Zellen gegenüber Apoptose-induzierenden chemotherapeutischen Agenzien umfasst;

Bestimmen einer oder mehrerer klinischer Faktoren des Patienten ausgewählt aus der Gruppe bestehend aus Alter, Cytogenetischer Satus, Leistung, histologische Unterklasse, Geschlecht und Krankheitszustand, und Klassifizierung des Patienten nach der Wahrscheinlichkeit einer klinischen Reaktion auf eine oder mehrere Krebsbehandlungen,

wobei die Wahrscheinlichkeit der klinischen Reaktion bestimmt wird durch die Beurteilung eines Prozent Primings, wobei das Prozent Priming die Reaktion des Krebszellspezimens auf jenes Kontaktieren mit einem oder mehreren BH3 Domänen-Peptiden misst und das Prozent Priming durch die folgende Gleichung definiert ist:

$$\%Priming = \left[ 100 * \left( \frac{DMSO\ AUC - Peptid_1\ AUC}{DMSO\ AUC - CCCP_{avg}\ AUC} \right) \right] Peptid_1$$

$$+ \left[ 100 * \left( \frac{DMSO\ AUC - Peptid_2\ AUC}{DMSO\ AUC - CCCP_{avg}\ AUC} \right) \right] Peptid_2$$

$$+ \cdots / (n\ Peptide)$$

wobei:

AUC entweder

(i) der Bereich unter der Kurve einer homogenen Zeitaufgelösten Fluoreszenz (HTRF) Messung ist, oder
(ii) die durchschnittliche Signalintensität einer fluoreszenzaktivierten Zellsortierungs- (FACS) Messung zu einem einzelnen Zeitpunkt ist, der zwischen etwa 5 min und etwa 300 min nach dem Start jenes Kontaktierens liegt,
jener Veränderung im Mitochondrialen Membranpotenzial ist;

DMSO (Dimethylsulfoxid) eine negative Baseline Kontrolle ist; und
CCCP (Carbonylcyanid-*m*-chlorophenylhydrazon) ein chemischer Inhibitor der oxidativen Phosphorylierung ist und durch das Dienen als Entkopplungs-Agens des Protonengradienten, der während der normalen Aktivität der Elektronenträger in der Elektronentransportkette in den Mitochondrien aufgebaut worden ist, einen Effektor der Proteinsynthese umfasst; und eine positive Baseline Kontrolle ist;
das Peptid jenes eine oder mehrere der BH3 Domänenn-Peptide ist;

wobei der eine oder die mehreren klinischen Faktoren ausgewählt sind, um die Spezifität und/ oder Sensitivität des BH3 Profils zur Assoziation mit der klinischen Reaktion zu erhöhen.

2. Das Verfahren nach Anspruch 1, wobei die Krebsbehandlung eine oder mehrere von ein BH3 Mimetikum, ein epigenetisch modifizierendes Agens, ein Topoisomerase Inhibitor, ein Modulator der Zellzyklusregulation und ein Kinesin-Spindel Protein stabilisierendes Agens ist.

3. Das Verfahren nach Anspruch 2, wobei der Modulator der Zellzyklusregulation ein Cyclin-abhängiger Kinase Inhibitor ist.

4. Das Verfahren nach Anspruch 1, wobei das Bestimmen des BH3 Profils die Verwendung des BH3 Domänen-Peptids umfasst, wobei das Peptid eines oder mehrere von BIM, BIM2A, BAD, BID, HRK, PUMA, NOXA, BMF, BIK und PUMA2A ist.

**5.** Das Verfahren nach Anspruch 1, ferner umfassend das Messen des BH3 Profils und einem oder mehrere von einem Zelloberflächenmarker CD33, einem Zelloberflächenmarker CD34, einem FLT3 Mutationsstatus, einem p53 Mutationsstatus, einem Phosphorylierungsstatus von MEK-1 Kinase und Phosphorylierung von Serin an Position 70 von Bcl-2; und Korrelieren zur Effizienz bei der Behandlung von AML Patienten mit Cytarabin oder Cytarabin-basierter Chemotherapie und/ oder Azacytidin.

**Revendications**

**1.** Procédé pour déterminer un traitement du cancer pour un patient, comprenant :

la détermination d'un profil BH3 pour un spécimen de cellules cancéreuses du patient, dans lequel le cancer est choisi parmi une leucémie myélogène aiguë, ou un myélome multiple, et dans lequel la détermination du profil BH3 comprend la perméabilisation les cellules cancéreuses du patient, la détermination d'un changement dans le potentiel de membrane mitochondriale lors de la mise en contact des cellules perméabilisées avec un ou plusieurs peptides de domaine BH3 ; et la corrélation d'une perte de potentiel de membrane mitochondriale avec une chimiosensibilité des cellules à des agents chimiothérapeutiques induisant l'apoptose ;
la détermination d'un ou plusieurs facteurs cliniques du patient choisis dans le groupe consistant en l'âge, le statut cytogénétique, la performance, la sous-classe histologique, le genre, et le stade de la maladie, et
la classification du patient quant à la probabilité d'une réponse clinique à un ou plusieurs traitements du cancer, dans lequel la probabilité de la réponse clinique est déterminée en estimant une sensibilisation en pour cent, dans lequel la sensibilisation en pour cent mesure la réponse du spécimen de cellules cancéreuses à ladite mise en contact avec un ou plusieurs peptides de domaine BH3 et la sensibilisation en pour cent est définie par l'équation suivante :

$$\%Sensibilisation = \left[ 100 \cdot \left( \frac{SSC\ DMSO\ -\ SSC\ Peptide_1}{SSC\ DMSO\ -\ SSC\ CCCP_{avg}} \right) \right] Peptide_1 + \left[ 100 \cdot \left( \frac{SSC\ DMSO\ -\ SSC\ Peptide_2}{SSC\ DMSO\ -\ SSC\ CCCP_{avg}} \right) \right] Peptide_2 + \cdots / (n\ peptides)$$

dans lequel :

SSC est soit

(i) la surface sous une courbe d'une mesure par fluorescence à résolution temporelle homogène (HTRF), soit
(ii) l'intensité de signal moyenne d'une mesure par cytométrie en flux (CMF) en un seul instant qui se produit entre environ 5 min et environ 300 min après le début de ladite mise en contact,

dudit changement dans le potentiel de membrane mitochondriale ;
DMSO (diméthyl sulfoxyde) est un témoin négatif de ligne de base ; et
CCCP (carbonylcyanure *m*-chlorophénylhydrazone) est un inhibiteur chimique de phosphorylation oxydative et comprend un effecteur de synthèse de protéines en servant d'agent de découplage du gradient de protons établi pendant l'activité normale de porteurs d'électrons dans la chaîne de transport d'électrons dans les mitochondries, et est un témoin positif de ligne de base ;
Peptide est lesdits un ou plusieurs peptides de domaine BH3 ;
dans lequel les un ou plusieurs facteurs cliniques sont choisis pour augmenter la spécificité et/ou la sensibilité du profil BH3 pour association avec une réponse clinique.

**2.** Procédé selon la revendication 1, dans lequel le traitement du cancer est un ou plusieurs parmi une substance mimétique de BH3, un agent modificateur épigénétique, un inhibiteur de topoisomérase, un modulateur de la régulation du cycle cellulaire, et un agent stabilisateur de protéines de fuseau-kinésine.

**3.** Procédé selon la revendication 2, dans lequel le modulateur de la régulation du cycle cellulaire est un inhibiteur de kinases dépendantes des cyclines.

**4.** Procédé selon la revendication 1, dans lequel la détermination du profil BH3 comprend l'utilisation du peptide de domaine BH3, dans lequel le peptide est un ou plusieurs parmi BIM, BIM2A, BAD, BID, HRK, PUMA, NOXA, BMF, BIK, et PUMA2A.

**5.** Procédé selon la revendication 1, comprenant en outre la mesure du profil BH3 et un ou plusieurs parmi un marqueur de surface cellulaire CD33, un marqueur de surface cellulaire CD34, un statut de mutation de FLT3, un statut de mutation de p53, un état de phosphorylation de MEK-1 kinase, et une phosphorylation de sérine en position 70 de Bcl-2 ; et la corrélation à l'efficacité dans le traitement de patients atteints de LAM avec la cytarabine ou une chimiothérapie à base de cytarabine et/ou l'azacytidine.

**MRL-14**

FIG. 1

FIG 1, continued

FIG 1, continued

EP 3 236 262 B1

EP 3 236 262 B1

FIG. 1, continued

FIG. 2

## MRL1

## MRL2

FIG 3

FIG. 3, continued

MRL5

MRL6

FIG. 3. continued

## MRL7

## MRL8

FIG. 3, continued

## MRL-9

## MRL-10

FIG. 4, continued

## MRL-11

## MRL-12

FIG. 4, continued

## MRL-13

## MRL-14

FIG. 4 continued

FIG 4, continued

FIG. 4 continued

| Cell Line | Therapeutic Activity | L-BIM_suspension |
|---|---|---|
| DHL10 | +, + | 4.6 |
| L | +, + | 32.3 |
| K | +, + | 47.1 |
| I | +, + | 50.0 |
| M | ++, ++ | 69.5 |
| H929 | +++, +++ | 75.5 |
| DHL6 | +++, +++ | 83.4 |
| J | +++, +++ | 84.5 |
| N | +++, +++ | 90.1 |

| Cell Line | Therapeutic Activity | L-BIMPUMA_suspension |
|---|---|---|
| DHL10 | +, + | 3.1 |
| L | +, + | 16.8 |
| K | +, + | 27.8 |
| I | +, + | 36.5 |
| M | ++, ++ | 44.0 |
| H929 | +++, +++ | 45.5 |
| J | +++, +++ | 54.1 |
| DHL6 | +++, +++ | 61.3 |
| N | +++, +++ | 65.2 |

BH3 Profiling vs Low-BIM_Suspension Lines

BH3 Profiling vs Low-BIM+PUMA_Suspension Lines

FIG. 5

EP 3 236 262 B1

FIG. 6

| Cell Line | Ther Act | PUMA_adherent |
|---|---|---|
| G | +, + | 26.2 |
| B | +, ++ | 35.1 |
| F | ++, ++ | 40 |
| A | ++, ++ | 45.9 |
| E | +++, ++++ | 37.5 |
| C | +++, ++++ | 75 |
| D | ++++, ++++ | 62.2 |
| H | ++++, ++++ | 69.4 |

BH3 Profiling vs PUMA_Adherent Lines

%PUMA

Therapeutic Activity

EP 3 236 262 B1

FIG. 6, continued

## BH3 Profiling vs NOXA_Adherent Lines

| Cell Line | Ther Act | NOXA_adherent |
|-----------|----------|---------------|
| G | +, + | 15.8 |
| B | +, ++ | 20.2 |
| F | ++, ++ | 15.0 |
| A | ++, ++ | 20.7 |
| E | +++, ++++ | 21.6 |
| C | +++, ++++ | 46.7 |
| D | ++++, ++++ | 37.6 |
| H | ++++, ++++ | 59.5 |

Therapeutic Activity

FIG. 6, continued

| Cell Line | Ther Act | L_BIMNOXA adherent |
|---|---|---|
| G | +, + | 9.4 |
| B | +, ++ | 10.1 |
| F | ++, ++ | 9.8 |
| A | ++, ++ | 17.3 |
| E | +++, ++++ | 23 |
| C | +++, ++++ | 46.1 |
| D | ++++, ++++ | 36.7 |
| H | ++++, ++++ | 63.7 |

BH3 Profiling vs Low-BIM+NOXA_Adherent Lines

FIG. 6, continued

EP 3 236 262 B1

FIG. 7

NOXA_Suspension

BAD_Suspension

$R^2 = 0.804$

$R^2 = 0.576$

FIG. 7, continued

EP 3 236 262 B1

FIG. 7, continued

BIM+PUMA+NOXA+HRK+BAD_Suspension

BIM+PUMA+NOXA+HRK_Suspension

$R^2$=0.913

$R^2$=0.890

% priming (sum)

% priming (sum)

EU-5148 EC$_{50}$ (uM)

EU-5148 EC$_{50}$ (uM)

FIG. 8

FIG. 8, continued

**BIM+PUMA+NOXA_Suspension**

**BIM+PUMA_Suspension**

$R^2=0.852$

$R^2=0.723$

FIG. 8, continued

FIG. 9

## NOXA_Adherent

$R^2=0.874$

% Priming (NOXA)

EU-5148 EC$_{50}$ (uM)

## BAD_Adherent

$R^2=0.295$

% Priming (BAD)

EU-5148 EC$_{50}$ (uM)

FIG. 9, continued

## HRK_Adherent

$R^2=0.010$

% Priming (HRK)

EU-5148 $EC_{50}$ (uM)

FIG. 9, continued

BIM+PUMA_Adherent

BIM+NOXA_Adherent

$R^2=0.773$

$R^2=0.817$

% priming (sum)

EU-5148 EC$_{50}$ (uM)

FIG. 10

FIG. 10, continued

EP 3 236 262 B1

BIM+PUMA+NOXA+HRK_Adherent

BIM+PUMA+NOXA+HRK+BAD_Adherent

FIG. 10, continued

FIG. 11

BH3 Profiling Readout, BAD response

%Cell Death, ARRY-520 at 200 nM

ARRY520 response, MM/AML Cell Lines

FIG. 11, continued

FIG. 12

FIG. 12, continued

EP 3 236 262 B1

A

## Bim

p=0.0000018

%Priming

NR          CR

B.

## Bim :ROC curve

AUC=0.83

Sensitivity

Specificity%

FIG. 13

FIG. 14

FIG. 14, continued

FIG. 14, continued

FIG. 14, continued

FIG. 15

FIG. 16

FIG. 16, continued

E.

**HRK-intermediate cyto**

p=0.0055

F.

**HRK-intermediate cyto**

AUC=0.873

Specificity%

FIG. 16, continued

G.

H.

**BM-unfavorable cyto**

**BM-unfavorable cyto**

p=0.016

AUC=0.800

FIG. 16, continued

A:

FIG. 17

B.

**BH3 Profile:BIM(0.1)**

C.

**BH3 Profile:BIM(0.1)**

AUC=0.752

D.

**BCL2L11 Protein Level**

p=0.33

E.

**BCL2L11 Protein Level**

AUC=0.613

FIG. 17, continued

FIG. 18

FIG. 19

FIG. 19, continued

FIG. 19, continued

## BIM+PUMA

## NOXA+PUMA

## PUMA+HRK

## BIM+NOXA

FIG. 20

FIG. 21

FIG. 21, continued

FIG. 22

FIG. 22, continued

FIG. 23

FIG. 24

## PUMA+NOXA+HRK

FIG. 24, continued

EP 3 236 262 B1

All BH3 Priming Values - Mayo AML (n=22)

FIG. 25

FIG. 26

FIG. 26, continued

FIG. 26, continued

FIG. 26, continued

| | EC50 (uM) - 48h, 5% serum | | _ | BH3 Profiling (% priming relative to control) | | | | | | |
|---|---|---|---|---|---|---|---|---|---|---|
| | Therapeutic Activity | Control Cmpd | | BIM | L-BIM | PUMA | L-PUMA | NOXA | BAD | HRK |
| adherent | | | | | | | | | | |
| A | ++, ++ | 13.5 | | 76.6 | 13.8 | 45.9 | 11.6 | 20.7 | 70.6 | 21 |
| B | +, ++ | 10 | | 64.6 | 0 | 35.1 | 0 | 20.2 | 59.7 | 0 |
| C | +++, ++++ | 16 | | 96.2 | 45.5 | 75.0 | 13.3 | 46.7 | 53.6 | 16.3 |
| D | ++++, ++++ | 7.5 | | 98.6 | 35.7 | 62.2 | 0 | 37.6 | 36.7 | 10.1 |
| E | +++, ++++ | 12 | | 103.5 | 24.3 | 37.5 | 0 | 21.6 | 68.1 | 13.4 |
| F | ++, ++ | 6 | | 91.8 | 4.5 | 40.0 | 0.5 | 15.0 | 85.2 | 13.6 |
| G | +, + | 18.5 | | 90.5 | 2.8 | 26.2 | 0 | 15.8 | 45.2 | 1.6 |
| H | ++++, ++++ | 14 | | 101.3 | 68.6 | 69.4 | 9.8 | 59.5 | 46.1 | 18.5 |

**TABLE 1**

| Suspension | EC50 (uM) - 48h, 5% serum | | | | | | | | | |
|------------|------|------|------|------|------|------|------|------|------|------|
| I | +, + | 2 | | 87.5 | 50.0 | 77.5 | 22.9 | 46.2 | 87.0 | 65.9 |
| J | +++, +++ | 1.7 | | 99.0 | 84.5 | 89.3 | 23.6 | 64.4 | 79 | 58.2 |
| K | +, + | 0.6 | | 96.9 | 47.1 | 88.1 | 8.5 | 14.7 | 63.1 | 14.7 |
| L | +, + | 2 | | 96.3 | 32.3 | 81.0 | 1.3 | 21.5 | 52.9 | 1.8 |
| M | ++, ++ | <0.4 | | 98.1 | 69.5 | 92.9 | 18.4 | 14.9 | 95.3 | 5.9 |
| N | +++, +++ | <0.4 | | 99.3 | 90.1 | 96.7 | 40.2 | 65.0 | 97.7 | 67.0 |
| DHL10 | + | 20 uM | | 30.1 | 4.6 | 10.3 | 1.6 | 19.1 | 24 | 0.8 |
| DHL6 | +++ | 0.4 uM | | 100.8 | 83.4 | 91.3 | 39.2 | 59 | 84.6 | 61.7 |
| H929 | +++ | 10 uM | | 97.2 | 75.5 | 83.8 | 15.4 | 82.1 | 46.1 | 36.6 |

**TABLE 1 (CONT.)**

| | $R^2$ Values (% Priming versus $EC_{50}$) | | |
|---|---|---|---|
| | suspension | adherent | mean |
| BIM | 0.626 | 0.731 | 0.679 |
| PUMA | 0.767 | 0.736 | 0.752 |
| NOXA | **0.804** | **0.874** | **0.839** |
| BAD | 0.575 | 0.295 | 0.435 |
| HRK | 0.775 | 0.010 | 0.393 |
| BIM+PUMA | 0.723 | 0.773 | 0.748 |
| BIM+NOXA | **0.838** | **0.817** | **0.828** |
| NOXA+PUMA | **0.874** | **0.813** | **0.844** |
| BIM+PUMA+NOXA | **0.852** | **0.811** | **0.832** |
| BIM+PUMA+NOXA+HRK | **0.890** | 0.571 | 0.731 |
| BIM+PUMA+NOXA+HRK+BAD | **0.913** | 0.482 | 0.698 |

$R^2$ values > 0.8 denoted in
**bold**-type

TABLE 2

| | | Responders (n=40) | Non-Responders (n=22) | p-value |
|---|---|---|---|---|
| Age | Mean (SD) | 53.1 (15.0) | 63.3 (12.7) | 0.008 |
| Gender | Male | 16 | 11 | 0.55 |
| | Female | 24 | 12 | |
| FAB | M0 | 0 | 2 | 0.29 |
| | M1 | 7 | 2 | |
| | M2 | 9 | 9 | |
| | M4 | 10 | 6 | |
| | M5 | 11 | 2 | |
| | M7 | 1 | 0 | |
| | RAEBT | 1 | 2 | |
| Cytogenetics | Favorable | 4 | 0 | 0.006 |
| | Intermediate | 26 | 8 | |
| | Unfavorable | 9 | 15 | |
| Ras mutation | Positive | 4 | 4 | 0.44 |
| | Negative | 19 | 13 | |
| Performance Status | 0 | 4 | 8 | 0.11 |
| | 1 | 22 | 12 | |
| | 2+ | 14 | 3 | |
| FLT3 | ITD or D835 Mutation | 12 | 7 | 0.97 |
| | No ITD or D835 mutation | 28 | 16 | |
| Source of Biopsied material | Bone marrow | 14 | 7 | 0.65 |
| | PBMC | 24 | 16 | |

TABLE 3

| BH3 Peptide | Mean %Priming +/– SD | | p–value* | AUC [95%CI] |
|---|---|---|---|---|
| | NR | CR | | |
| BIM | 70.1 ± 32.6 | 88.2 ± 17.6 | 0.023 | 0.68 [0.53,0.82] |
| BIM(0.1) | 13.8 ± 13.4 | 36.8 ± 21.2 | 0.0000018 | 0.83 [0.72,0.93] |
| PUMA | 44.7 ± 29.5 | 64.0 ± 22.7 | 0.017 | 0.69 [0.54,0.84] |
| PUMA(10) | 33.3 ± 24.3 | 50.4 ± 23.7 | 0.0064 | 0.71 [0.58,0.85] |
| NOXA | 26.3 ± 15.5 | 35.1 ± 24.9 | 0.20 | 0.60 [0.46,0.75] |
| BAD | 33.2 ± 29.6 | 52.7 ± 24.3 | 0.014 | 0.70 [0.55,0.85] |
| BMF | 45.6 ± 31.6 | 64.4 ± 24.9 | 0.016 | 0.69 [0.53,0.84] |
| HRK | 20.8 ± 22.4 | 36.6 ± 22.4 | 0.010 | 0.72 [0.57,0.88] |
| PUMA2A | 10.8 ± 19.5 | 16.1 ± 18.9 | 0.16 | 0.61 [0.45,0.78] |

\* Statistical significance established as p<0.01 by Mann–Whitney analysis

Table 4

| | Partition Analyses | Continuous Variable Analyses | |
|---|---|---|---|
| | p-values (Mann-Whitney) | R² values (log) | p-values |
| | -JAK2 (N=13) | -JAK2 (N=13) | |
| Bim | p=0.0011 | 0.37 | p=0.0278 |
| Puma | p=0.0046 | 0.70 | p=0.0004 |
| Noxa | p=0.022 | 0.27 | p=0.0662 |
| Hrk | p=0.022 | 0.40 | p=0.0036 |
| Bad | p=1 | 0.14 | p=0.2012 |
| Puma2A | p=0.53 | 0.23 | p=0.0967 |
| BimPuma | p=0.0011 | 0.58 | p=0.0026 |
| BimNoxa | p=0.0011 | 0.37 | p=0.0261 |
| PumaNoxa | p=0.0011 | 0.64 | p=0.0011 |
| BimPumaNoxa | p=0.0011 | 0.56 | p=0.0063 |
| BimPumaNoxaHrk | p=0.0011 | 0.63 | p=0.001 |
| BimPumaNoxaHrkBad | p=0.0011 | 0.64 | p=0.0042 |
| PumaHrk | p=0.0011 | 0.85 | p<0.0001 |
| BimPumaHrk | p=0.0011 | 0.61 | p=0.0016 |
| PumaNoxaHrk | p=0.0011 | 0.70 | p=0.0003 |

Table 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20120225851 A
- WO 2012122370 A
- US 8168755 B
- US 7868133 B
- US 8221966 B
- US 20110130309 A

**Non-patent literature cited in the description**

- **THOMENIUS et al.** *Blood,* 2011, vol. 118 (21), 1690
- **SINICROPE et al.** *Clinical Cancer Research,* 2008, vol. 15, 4128-4133
- *Clinical Cancer Research,* 2008, vol. 14, 5810-5818
- **NI CHONGHAILE et al.** *Science,* 2011, vol. 334 (6059), 1129-1133
- *Blood,* 2011
- **BODET et al.** *British J. Cancer,* 2010, vol. 103 (12), 1808-1814
- **DEL GAIZO MOORE et al.** *J. Clin. Invest.,* 2007, vol. 117 (1), 112-121
- **AGNEW.** *Chem. Intl. Ed. Engl.,* 1994, vol. 33, 183-186
- Stapled Peptides for Intracellular Drug Targets. **VERDINE et al.** Methods in Enzymology. vol. 503
- **ELSTON ; ELLIS.** *Histopathology,* 1991, vol. 19, 403-10
- **VO et al.** *Cell,* 2012, vol. 151 (2), 344-355